# EUROPÄISCHE PATENTSCHRIFT

(11) **EP 4 465 917 B1**
(45) Veröffentlichungstag und Bekanntmachung des Hinweises auf die Patenterteilung: **04.03.2026**
(21) Anmeldenummer: 23701018.6
(22) Anmeldetag: 16.01.2023
(51) Int. Cl.: A61B 50/30, A61F 2/00

(54) **VERPACKUNG FÜR EIN MEDIZINISCHES PRODUKT**
PACKAGING FOR A MEDICAL PRODUCT
EMBALLAGE POUR UN PRODUIT MÉDICAL

(30) Priorität: 20.01.2022 DE 102022101267
(43) Veröffentlichungstag der Anmeldung: 27.11.2024
(73) Patentinhaber: Aesculap AG, 78532 Tuttlingen (DE)
(72) Erfinder: HUBER, Christian, 78570 Mühlheim (DE); OSSWALD, Nadine, 78532 Tuttlingen (DE)
(74) Vertreter: Winter, Brandl - Partnerschaft mbB
(86) Internationale Anmeldenummer: PCT/EP2023/050889
(87) Internationale Veröffentlichungsnummer: WO 2023/139028

(56) Entgegenhaltungen:
- EP-A2- 0 579 377
- US-A- 3 012 705
- US-A- 4 884 681
- US-A1- 2011 309 073
- US-A1- 2019 209 282
- US-B1- 10 966 815

## Beschreibung

Die vorliegende Offenbarung betrifft eine Verpackung für ein oder eines medizinischen Produkts bzw. für ein Medizinprodukt (nachfolgend auch als Produkt-Schutzverpackung bezeichnet) mit einer eine Sterilbarriere ausbildenden Umverpackung bzw. Sterilverpackung, die einen versiegelten oder versiegelbaren Aufnahmeraum für das medizinische Produkt bzw. Medizinprodukt ausbildet und einer Produktauflagerung und/oder Halterung zur kontaktfreien Lagerung des Produkts innerhalb der Umverpackung.

Medizinprodukte, insbesondere Implantate, werden häufig in den menschlichen oder tierischen Körper eingesetzt. Eine wichtige Anforderung an Medizinprodukte, vor allem an Implantate, ist daher, dass sie steril sind, um Infektionen durch verunreinigte Medizinprodukte im menschlichen oder tierischen Körper zu verhindern. Um sicherzugehen, dass Medizinprodukte steril sind und bleiben, werden sie, üblicherweise vom Hersteller, sterilisiert bzw. unter Sterilbedingungen hergestellt. Die sterilen Medizinprodukte werden dann in eine Sterilverpackung (bzw. Umverpackung) eingeführt. Die Sterilverpackung weist eine Sterilbarriere auf, die das Eindringen von Verunreinigungen und einen Austausch zwischen ihrem Inneren und der Umgebung verhindert. Anschließend wird die Sterilverpackung, die das Medizinprodukt umschließt, vakuumiert. Wenn das Medizinprodukt in der vakuumierten Sterilverpackung transportiert wird, kann ausgeschlossen werden, dass das Medizinprodukt von außerhalb der Sterilverpackung verunreinigt wird. Bei Bedarf kann das derart steril verpackte und transportierte Medizinprodukt, insbesondere Implantat, aus der Sterilverpackung ausgepackt und in einen menschlichen oder tierischen Körper eingesetzt werden.

Als Sterilverpackungen werden zumeist vakuumierbare Sterilbeutel verwendet. Insbesondere dann, wenn das Medizinprodukt eine raue Oberfläche hat, was vor allem bei Hüft- und Knieimplantaten der Fall ist, besteht die Gefahr, dass sich aufgrund der rauen Medizinproduktoberfläche beim Einführen des Medizinprodukts in die Sterilverpackung und/oder beim Transportieren des in der Sterilverpackung aufgenommenen Medizinprodukts und/oder beim Entnehmen des Medizinprodukts aus der Sterilverpackung durch Abrieb Folienpartikel von dem Sterilbeutel bzw. der Sterilverpackung lösen. Diese Folienpartikel können sich auf dem Medizinprodukt absetzen und an diesem anhaften. Die an dem Medizinprodukt zurückbleibenden Folienpartikel können beim Einsetzen des Medizinprodukts in den tierischen oder menschlichen Körper auf diese Weise in den Körper gelangen. Dies kann die Gesundheit und das Wohlbefinden des entsprechenden Patienten (stark) beeinträchtigen.

Daher wurde bisher für Medizinprodukte, vor allem für solche mit rauer Oberfläche, eine formstabile Halterung bzw. Produkt-Schutzverpackung, v.a. aus (duroplastischem oder thermoplastischem) Kunststoff, bspw. aus PETG, bereitgestellt, welche selbst steril ist und das sterilisierte bzw. sterile Medizinprodukt umschließt. Die Produkt-Schutzverpackung mit dem darin angeordneten Medizinprodukt wird dann in die vakuumierbare Sterilverpackung eingeführt. Nach dem Vakuumieren der Sterilverpackung ist das so verpackte Medizinprodukt steril transportierbar. Allerdings kann es sein, dass die formstabilen Produkt-Schutzverpackungen aufgrund des beim und durch das Vakuumieren auftretenden (starken) Unterdrucks brechen oder verbogen werden. Zudem führen formstabile Produkt-Schutzverpackungen dazu, dass sich an dem sie aufnehmenden Sterilbeutel bzw. Sterilverpackung viele Falten ausbilden.

Vor diesem Hintergrund offenbart US 10 966 815 B1 eine Umschließvorrichtung u.a. zum Transport von einer medizinischen Vorrichtung. Die Umschließvorrichtung ist sterilisierbar und kann aus einem Flachzuschnitt über eine Klappfalzlinie in eine nicht vollständig geschlossene Taschenform gefaltet werden.

EP 0 579 377 A2 offenbart eine Verpackung zur Aufnahme eines medizinischen Produkts, bspw. eines medizinischen Gewebes, um dieses Produkt während der Sterilisation zu schützen. Die Verpackung ist aus einem Flachzuschnitt entlang einer Falzlinie in eine nicht vollständig geschlossenen Taschenform faltbar.

US 2011 / 309073 A1 offenbart eine Verpackung zum indirekten Aufbewahren einer medizinischen Vorrichtung, wie bspw. eines chirurgischen Gewebes, die aus einem Flachzuschnitt durch Falten in eine Speicherform überführbar ist. Eine Mappe die unmittelbar die medizinische Vorrichtung in sich aufnimmt, ist in einer versiegelbaren Umverpackung 134 aufgenommen. Die Umverpackung ist von der Verpackung aufgenommen.

US 4 884 681 A offenbart eine Haltevorrichtung bzw. Produktauflagerung für chirurgische Fäden und Nadeln. Die Haltevorrichtung ist durch Falten eines Flachzuschnittes entlang der Klapplinie in eine Taschenform zur Aufnahme des chirurgischen Materials überführbar. Die Haltevorrichtung kann in einem sterilen, hermetisch dichten / versiegelten Umschlag bzw. Umverpackung aufgenommen werden.

US 3 012 705 A offenbart einen Flachzuschnitt für das Falten einer Schachtel, die nicht explizit für die Aufnahme von medizinischen Produkten vorgesehen ist und offenbart dabei keine Umverpackung für die aus dem Flachzuschnitt gebildete Schachtel.

US 2019 / 209282 A1 offenbart eine Verpackung zur Aufnahme einer flächigen medizinischen Vorrichtung (z.B. chirurgisches Gewebe), die aus einem Flachzuschnitt faltbar ist und offenbart dabei keine Umverpackung für die aus dem Flachzuschnitt gebildete Verpackung.

Es ist somit eine Aufgabe der vorliegenden Offenbarung, die Probleme aus dem Stand der Technik zu vermindern oder zu beseitigen. Insbesondere soll eine Verpackung für ein medizinisches Produkt (Produkt-Schutzverpackung) mit einer eine Sterilbarriere ausbildenden Umverpackung, die einen versiegelten oder versiegelbaren, insbesondere vakuumierten, Aufnahmeraum für das medizinische Produkt ausbildet und einer Produktauflagerung und/oder Halterung zur kontaktfreien Lagerung des Produkts innerhalb der Umverpackung bereitgestellt werden, deren Produktauflagerung trotz großer auf sie einwirkender Kräfte nicht bricht oder verbogen wird. Außerdem soll die entsprechende Produktauflagerung verhindern, dass die sie aufnehmende, insbesondere vakuumierte, Umverpackung bzw. Sterilverpackung viele Falten bildet.

Diese Aufgabe wird durch die offenbarungsgemäße Verpackung (Produkt-Schutzverpackung) nach dem Anspruch 1 gelöst.

Offenbarungsgemäß ist eine Verpackung für ein oder eines medizinischen Produkts, insbesondere eines sterilen Medizinprodukts, vorzugweise Implantat mit rauer Oberflächenstruktur (Produkt-Schutzverpackung), mit einer eine Sterilbarriere ausbildenden Umverpackung oder Sterilverpackung, die einen versiegelten oder versiegelbaren, insbesondere vakuumierten, Aufnahmeraum für das medizinische Produkt ausbildet und einer Produktauflagerung und/oder Halterung zur kontaktfreien Lagerung des Produkts innerhalb der Umverpackung vorgesehen. Dabei ist die Produktauflagerung und/oder Halterung eine, vorzugsweise taschenförmige, Schutzhülle zur Aufnahme des Produkts und zur Beabstandung der Umverpackung vom Produkt, die aus einem einzigen, flexiblen Flachzuschnitt durch Falten (bzw. Klappen) entlang zumindest einer vordefinierten Klappfalzlinie hergestellt ist.

Eine derartige Verpackung hat den Vorteil, dass die, insbesondere taschenförmige, Schutzhülle, die das Medizinprodukt aufnimmt, dieses vor Folienpartikeln der Umverpackung bzw. Sterilverpackung schützt. Außerdem kann die derartige Schutzhülle großen Kräften, wie sie bspw. beim und durch das Vakuumieren in der Umverpackung bzw. Sterilverpackung vorliegen, standhalten. Die Handhabung der Schutzhülle, insbesondere das Hinein- und Herausführen der Schutzhülle in die bzw. aus der Umverpackung bzw. Sterilverpackung, ist besonders einfach und angenehm. Grundsätzlich ist das in der (taschenförmigen) Schutzhülle aufgenommene Medizinprodukt mithilfe der Schutzhülle einfach in die Umverpackung bzw. Sterilverpackung einzuführen, darin zu positionieren und aus der Umverpackung bzw. Sterilverpackung zu entnehmen.

Da die Schutzhülle aus einem flexiblen Material ausgebildet ist, bricht die Schutzhülle auch unter großem Druck, wie z.B. durch den Unterdruck beim bzw. durch das Vakuumieren der die Schutzhülle aufnehmenden Umverpackung bzw. Sterilverpackung, nicht und verbiegt sich auch nicht irreversibel. Da das Material der Schutzhülle flexibel ist, schmiegt es sich durch den Unterdruck beim Vakuumieren der Umverpackung bzw. Sterilverpackung und in der vakuumierten Umverpackung bzw. Sterilverpackung an die Formgebung des durch sie aufgenommenen Medizinprodukts an und verursacht nur einen geringen Faltenwurf an der Umverpackung bzw. Sterilverpackung. Da die Schutzhülle aus einem Flachzuschnitt in eine Taschenform gefaltet ist, ist die Schutzhülle leicht und günstig zu fertigen. Für den Fall, dass der Flachzuschnitt ausschließlich durch Falten in die, insbesondere taschenförmige, Schutzhülle überführt ist, werden keine Bindemittel, wie bspw. Kleber an der Schutzhülle benötigt. Damit ist eine Gefahr, dass sich die Bindemittel vom Material der Schutzhülle lösen und am Medizinprodukt ablagern könnten, nicht gegeben. Die Schutzhülle kann zudem wieder aufgefaltet werden, um das Medizinprodukt einfach und mit minimierter Reibung von der Schutzhülle entnehmen zu können. Ein Abrieb von Partikeln aus dem Material der Schutzhülle ist damit minimiert.

Erfindungsgemäß weist der Flachzuschnitt eine als Auflage für das medizinische Produkt dienende Basisplatte auf, die eine Basis-Eingriffseinrichtung hat, und weist eine Klapp- oder Deckplatte auf, die mittels der Klappfalzlinie mit der Basisplatte gekoppelt ist und in einer in Richtung zur Basisplatte umgeklappten Position mit der Basis-Eingriffseinrichtung zum Halten der umgeklappten Position in Wirkeingriff bringbar ist, sodass die in einem Geschlossenzustand aufeinanderliegenden Stirnseiten von Basisplatte und Klapp- oder Deckplatte, die der Klappfalzlinie jeweils gegenüberliegen, verschlossen sind. Weiter ist erfindungsgemäß vorgesehen, dass die Schutzhülle in einem durch Falten erzeugten Geschlossenzustand nach allen Seiten hin zur Umverpackung hin verschlossen ist.

Vorteilhafte Aspekte der offenbarungsgemäßen Verpackung (Produkt-Schutzverpackung) sind Gegenstand der Unteransprüche und werden nachstehend erläutert.

Erfindungsgemäß ist die, insbesondere taschenförmige, Schutzhülle in einem durch Falten erzeugten Geschlossen-Zustand nach allen Seiten hin zur Umverpackung hin verschlossen.

Auf diese Weise kann das in der (taschenförmigen) Schutzhülle angeordnete Medizinprodukt nicht einfach aus der Schutzhülle rutschen oder fallen. Somit besteht keine Gefahr, dass das Medizinprodukt bspw. in die Umverpackung rutscht und sich dort dann Sterilverpackungspartikel durch Abrieb am Medizinprodukt ablagern. Die durch das Falten des Flachzuschnitts in Taschenform ausgebildete Schutzhülle sorgt also dafür, dass das von ihr umschlossene Medizinprodukt zu keinem Zeitpunkt unabsichtlich aus der Schutzhülle herausfällt oder herausrutscht.

Weiter ist es vorteilhaft, wenn der einzige Flachzuschnitt aus mehreren Einzelteilen hergestellt ist, welche miteinander zu dem einzigen Flachzuschnitt verschweißt, vernäht und/oder verklebt sind.

Dies hat den Vorteil, dass der Flachzuschnitt in Dimension und Form besonders individuell und modular an ein darin aufzunehmendes Medizinprodukt anpassbar ist.

Insbesondere ist es sinnvoll, wenn in dem Geschlossenzustand die einander jeweils gegenüberliegenden, insbesondere übereinanderliegenden, Seitenkanten der Basisplatte und der Deckplatte stoffschlüssig oder formschlüssig miteinander verbunden sind (abgesehen von den Seitenkanten, an denen die Klappfalzlinie angeordnet ist).

Auf diese Weise kann jede Seite der Schutzhülle im Geschlossenzustand einfach und sicher verschlossen werden. Damit kann verhindert werden, dass das Medizinprodukt über eine der Seiten aus der Schutzhülle entweicht.

Eine Ausführungsform der Schutzhülle bezieht sich darauf, dass der Flachzuschnitt folgendes aufweist: eine als Auflage für das medizinische Produkt dienende Basisplatte, die eine Basis-Eingriffseinrichtung hat, eine Klapp- oder Deckplatte (nachfolgend auch einfach als Deckplatte bezeichnet), die mittels einer Klappfalzlinie mit der Basisplatte gekoppelt ist und in einer in Richtung zur Basisplatte umgeklappten Position mit der Basis-Eingriffseinrichtung zum Halten der umgeklappten Position in Wirkeingriff bringbar ist, und eine erste Seitenplatte, die über eine senkrecht zur Klappfalzlinie ausgerichtete Schwenkfalzlinie mit der Basisplatte gekoppelt ist und eine Seiten-Eingriffseinrichtung zum Halten der ersten Seitenplatte in einer zur Basisplatte hin geschwenkten Position hat.

Wenn die Schutzhülle eine Basisplatte aufweist, auf die im Geschlossenzustand eine Deckplatte geklappt bzw. gefaltet ist (d.h. auf der die Deckplatte aufliegt), bilden Basisplatte und Deckplatte in Kooperation ein Hauptfach für die Aufnahme eines Medizinprodukts aus. Auf diese Weise kann ganz einfach durch Falten eines Flachzuschnitts mit zwei Seiten, nämlich Deckplatte und Basisplatte, die Schutzhülle ausgebildet werden. Die Seite, an der Deckplatte und Basisplatte die gemeinsame Seitenkante und damit zwischen sich die Klappfalzlinie aufweisen, ist die taschenförmige Schutzhülle immer verschlossen. Über die Seitenplatte kann eine weitere Seitekante der Basisplatte und ggf. der Deckplatte verschlossen werden. Über den Wirkeingriff zwischen der Basis-Eingriffsvorrichtung der Basisplatte und der Deckplatte werden die entsprechenden Seitenkanten der Basisplatte und Deckplatte miteinander verbunden und somit verschlossen.

Es ist außerdem möglich, dass die Basisplatte zwei parallel zueinander verlaufende und voneinander beabstandete Aufnahmeschlitze aufweist. Diese können dazu ausgebildet sein, kooperativ ein Medizinprodukt oder Packmittel, z.B. ein Trockenmittel, an der Basisplatte zu befestigen.

Ist z.B. ein Medizinprodukt lose auf die Basisplatte aufgelegt und ein Produktmittel in den Aufnahmeschlitzen fixiert, verhindern die Aufnahmeschlitze, dass das Produkt während eines Transports der Schutzhülle innerhalb der Schutzhülle seine Position ändert.

Außerdem kann es vorgesehen sein, dass der Flachzuschnitt weiterhin eine zweite Seitenplatte aufweist, die über eine senkrecht zur Klappfalzlinie ausgerichtete Schwenkfalzlinie mit der Basisplatte gegenüberliegend zur ersten Seitenplatte gekoppelt ist und eine Seiten-Eingriffseinrichtung zum Halten der zweiten Seitenplatte in einer zur Basisplatte hin geschwenkten Position hat.

Mithilfe der zweiten Seitenplatte können alle Seiten der Schutzhülle verschlossen werden. Denn im Geschlossenzustand verschließt die Klappfalzlinie die eine Seite der Schutzhülle, insbesondere eine Stirnseite der Schutzhülle. Die Basis-Eingriffseinrichtung befindet sich vorteilhafterweise an der im Geschlossenzustand der Klappfalzlinie gegenüberliegenden Seite der Schutzhülle, insbesondere die andere Stirnseite der Schutzhülle. Der Wirkeingriff zwischen der Basis-Eingriffsvorrichtung der Basisplatte und der Deckplatte verschließt damit die andere Stirnseite der Schutzhülle. Die beiden anderen Seiten der Schutzhülle sind über die Seitenplatten verschlossen.

Es ist dabei insbesondere vorgesehen, dass die jeweilige Seiten-Eingriffsvorrichtung der ersten Seitenplatte und der zweiten Seitenplatte als Einrastschlitz ausgebildet ist, über den die erste Seitenplatte und die zweite Seitenplatte miteinander verhakbar sind, oder die jeweilige Seiten-Eingriffsvorrichtung der ersten Seitenplatte und der zweiten Seitenplatte als, insbesondere schlitzförmige, Aufnahme ausgebildet ist, in welche ein laschenartiger Abschnitt der Klapp- oder Deckplatte einführbar ist.

Durch das Verhaken oder über die Aufnahme können die erste und die zweite Seitenplatte formschlüssig und damit besonders sicher miteinander bzw. mit der Deckplatte verbunden werden. Dies verhindert, dass das medizinische Produkt bzw. Medizinprodukt einfach aus der in den Geschlossezustand gefalteten Schutzhülle herausrutschen oder herausfallen kann.

Es ist insbesondere vorteilhaft, wenn die erste und die zweite Seitenplatte jeweils einen Überlappungsbereich aufweist, mit dem sie sich im Geschlossenzustand gegenseitig überlappen. In dem Überlappungsbereich jeder Seitenplatte kann dann der Einrastschlitz vorgesehen sein, der dazu vorgesehen ist, im Geschlossenzustand mit dem Einrastschlitz des Überlappungsbereichs der anderen Seitenplatte verhakt zu sein. Alternativ zu dem Einrastschlitz ist im Überlappungsbereich einer Seitenplatte die Aufnahme vorgesehen, die im Geschlossenzustand über der Aufnahme des Überlappungsbereichs der anderen Seitenplatte liegt und eine durchgängige Aufnahme zur Aufnahme des laschenförmigen Abschnitts der Deckplatte ausbildet.

Auf beide Arten können die Seitenplatten sicher miteinander verbunden werden und vor einem zufälligen Öffnen geschützt im Geschlossenzustand gehalten werden.

Eine andere Ausführungsform der Schutzhülle sieht vor, dass der Flachzuschnitt folgendes aufweist: eine als Auflage für das medizinische Produkt dienende Basisplatte, die eine Basis-Eingriffseinrichtung hat, eine Klapp- oder Deckplatte, die mittels einer Klappfalzlinie mit der Basisplatte gekoppelt ist und in einer in Richtung zur Basisplatte umgeklappten Position mit der Basis-Eingriffseinrichtung zum Halten der umgeklappten Position in Wirkeingriff bringbar ist, eine Schließlasche, die über eine parallel zur Klappfalzlinie ausgerichtete Schwenkfalzlinie mit der Basisplatte oder der Klapp- oder Deckplatte gekoppelt ist, und eine Einstecklasche, die über eine parallel zur Klappfalzlinie ausgerichtete Schwenkfalzlinie mit derjenigen aus Basisplatte und Klapp-oder Deckplatte gekoppelt ist, die nicht mit der Schließlasche gekoppelt ist, und zwischen sich und der mit ihr gekoppelten aus Basisplatte oder Klapp- oder Deckplatte eine, insbesondere schlitzförmige, Laschenaufnahme zum Aufnehmen der Schließlasche ausbildet.

Diese Ausführungsform der Schutzhülle ist besonders einfach. Denn Seitenplatten sind hier zum Verschließen der Seitenkanten eines Seitenkantenpaars nicht erforderlich. Vielmehr sind zwei im Geschlossenzustand gegenüberliegende, insbesondere übereinanderliegende, Seitenkanten von Basisplatte und Deckplatte durch den Wirkeingriff der Basis-Eingriffseinrichtung und der Deckplatte nach außen hin verschlossen. Die im Geschlossenzustand von der Klappfalzlinie parallel beabstandeten Seitenkanten von Basisplatte und Deckplatte sind über die in die Laschenaufnahme eingreifende Schließlasche miteinander verbunden. Im Geschlossenzustand greift dann die Schließlasche formschlüssig in die Laschenaufnahme ein bzw. hintergreift diese. Die übrigen im Geschlossenzustand einander gegenüberliegenden, insbesondere übereinanderliegenden, Seitenkanten können jeweils stoffschlüssig, z.B. durch Schweißen, Siegeln oder Kleben, miteinander verbunden sein. Weitere Laschen oder dergleichen können daher an diesen Seitenkanten entfallen. Damit ist der Flachzuschnitt sehr einfach ausgeführt und daher leicht und günstig zu fertigen.

Eine weitere Ausführungsform der Schutzhülle sieht vor, dass der Flachzuschnitt aufweist: eine als Auflage für das medizinische Produkt dienende Basisplatte, die eine Basis-Eingriffseinrichtung hat, eine Klapp- oder Deckplatte, die mittels einer Klappfalzlinie mit der Basisplatte gekoppelt ist und in einer in Richtung zur Basisplatte umgeklappten Position mit der Basis-Eingriffseinrichtung zum Halten der umgeklappten Position in Wirkeingriff bringbar ist, und jeweils eine erste Zusatzlasche an der Basisplatte und an der Klapp-oder Deckplatte, die an die zueinander benachbarten und senkrecht zur Klappfalzlinie verlaufenden Seitenkanten der Basisplatte bzw. der Klapp-oder Deckplatte angrenzen und im Geschlossenzustand zusammen ein Zusatzfach zur Aufnahme eines weiteren Produkts, insbesondere eines weiteren Medizinprodukts oder ein Medizinprodukt begleitendes Packmittel, ausbilden.

Vorteilhafterweise ist das Zusatzfach räumlich von dem von Deckplatte und Basisplatte im Geschlossenzustand ausgebildeten Hauptfach (durch einen stoffschlüssigen Bereich) abgetrennt. Damit kann ein weiteres Medizinprodukt oder ein mit dem Medizinprodukt zu transportierendes Packmittel, z.B. ein Trockenmittel, in ein und derselben taschenförmigen Schutzhülle unter räumlicher Trennung angeordnet werden. Wenn auf diese Weise bspw. zwei Medizinprodukte, die insbesondere jeweils eine raue Oberfläche aufweisen, in der taschenförmigen Schutzhülle angeordnet sind, kann verhindert werden, dass sich die Medizinprodukte gegenseitig abstoßen und dabei gegenseitig Material abtragen.

Dabei ist es vorteilhaft, wenn der Flachzuschnitt weiterhin weitere zweite Zusatzlaschen, insbesondere jeweils zwei Zusatzlaschen, an der Basisplatte und an der Klapp- oder Deckplatte aufweist, die an eine von der senkrecht zur Klappfalzlinie verlaufenden Seitenkante der Basisplatte bzw. der Klapp- oder Deckplatte parallel beabstandete Seitenkante der ersten Zusatzlasche angrenzen und die zweiten Zusatzlaschen an der Basisplatte bzw. der Klapp- oder Deckplatte voneinander beabstandet sind und im Geschlossenzustand zusammen mit der über- bzw. unter ihr liegenden zweiten Zusatzlasche an der anderen aus Basisplatte und Klapp- oder Deckplatte weitere Zusatzfächer zur Aufnahme weiterer Produkte ausbilden.

Auf diese Weise können mehrere Medizinprodukte in ein und derselben taschenförmigen Schutzhülle räumlich getrennt voneinander transportiert werden.

Eine weitere Ausführungsform der Schutzhülle sieht vor, dass der Flachzuschnitt folgendes aufweist:
- eine als Auflage für das medizinische Produkt dienende Basisplatte, die eine Basis-Eingriffseinrichtung hat,
- eine Klapp- oder Deckplatte, die mittels der Klappfalzlinie mit der Basisplatte gekoppelt ist und in einer in Richtung zur Basisplatte umgeklappten Position mit der Basis-Eingriffseinrichtung zum Halten der umgeklappten Position in Wirkeingriff bringbar ist,
und die Klapp- oder Deckplatte in einen Verbindeabschnitt und in einen Aufreißabschnitt unterteilt ist, der über zumindest eine Perforationslinie von dem Verbindeabschnitt getrennt ist, die eine Sollbruchstelle zwischen Verbindeabschnitt und Aufreißabschnitt ausbildet.

Wenn ein derartiger Flachzuschnitt in die geschlossene Taschenform der Schutzhülle überführt ist, ermöglicht die Perforationslinie (als Sollbruchstelle) ein Aufreißen und damit ein Trennen des Aufreißabschnitts vom Verbindeabschnitt, der (im Schutzhüllenzustand der geschlossenen Taschenform) fest und stoffschlüssig mit der Basisplatte verbunden bleibt. Durch einfaches Aufbringen einer Zugkraft auf den Aufreißabschnitt kann dieser vom Verbindeabschnitt und damit auch von der restlichen Klapp- oder Deckplatte gelöst werden, sodass einfach und schnell eine Öffnung zur Entnahme eines in der Schutzhülle aufgenommenen Medizinprodukts freilegbar ist.

Vorzugsweise sind die in der umgeklappten Position einander gegenüberliegenden und sich senkrecht von der Klappfalzlinie aus erstreckenden (langen) Seitenkanten der Klapp- oder Deckplatte und der Basisplatte stoffschlüssig miteinander verbindbar bzw. in einem Zustand, in dem die Schutzhülle eine geschlossene Taschenform aufweist, miteinander verbunden.

Vorzugsweise grenzt im Falle nur einer Perforationslinie diese eine Perforationslinie den Aufreißabschnitt vollständig vom Verbindeabschnitt ab. Im Falle zweiter Perforationslinien schließen die beiden Perforationslinien den Aufreißabschnitt zwischen sich ein und trennen ihn auf diese Weise (vollständig) vom Verbindeabschnitt ab. Auf diese Weise grenzt /grenzen die Perforationslinie/n den Aufreißabschnitt deutlich und auf einfach realisierbare Weise vom Verbindeabschnitt ab.

Insbesondere sind im Falle zweiter Perforationslinien die beiden Perforationslinien so an der Klapp- oder Deckplatte angeordnet, dass der Aufreißabschnitt den Verbindeabschnitt in zwei voneinander separierte (ggf. gleichgroße) Hälften unterteilt. In anderen Worten ist der Aufreißabschnitt zwischen den beiden Hälften des Verbindeabschnitts angeordnet.

Es kann vorgesehen sein, dass ein erstes Ende der zumindest einen Perforationslinie und ein zweites Ende derselben Perforationslinie oder, im Falle zweier Perforationslinien an derselben Klapp- oder Deckplatte, eine erste Perforationslinie und eine zweite Perforationslinie in eine der Klappfalzlinie gegenüberliegende (kurze) Seitenkante der Klapp- oder Deckplatte münden. Mit anderen Worten wird der Aufreißabschnitt durch die zumindest eine Perforationslinie und die der Klappfalzlinie gegenüberliegende Seitenkante der Klapp- oder Deckplatte vollständig begrenzt.

Es ist zudem sinnvoll, wenn sich ausgehend von der der Klappfalzlinie gegenüberliegenden Seitenkante der Klapp- oder Deckplatte ein laschenartiger Abschnitt der Klapp- oder Deckplatte von der Klapp- oder Deckplatte weg erstreckt, der insbesondere mit der, vorzugsweise als Laschenaufnahme ausgebildeten, Basis-Eingriffseinrichtung an der Basisplatte in Wirkeingriff bringbar ist, und der stoffeinstückig mit dem Aufreißabschnitt ausgebildet ist. Der laschenartige Abschnitt der Deckplatte oder Basisplatte kann leicht von einem Verwender gegriffen werden. Durch Aufbringen einer Zug- bzw. Reißkraft auf den laschenartigen Abschnitt kann der Verwender einfach und schnell, vorzugsweise mit nur einem Handgriff, den Aufreißabschnitt entlang der Perforationslinie von dem Verbindeabschnitt und damit von der restlichen Klapp- oder Deckplatte entfernen (sodass einfach und schnell eine Öffnung zur Entnahme eines in der Schutzhülle aufgenommenen Medizinprodukts freilegbar ist).

Weiterhin ist denkbar, dass das erste Ende der zumindest einen Perforationslinie und das zweite Ende derselben Perforationslinie oder, im Falle zweier Perforationslinien an derselben Klapp- oder Deckplatte, die erste Perforationslinie und die zweite Perforationslinie an der Stelle, an der sie jeweils in die der Klappfalzlinie gegenüberliegende (kurze) Seitenkante der Klapp- oder Deckplatte münden, um die Breite des laschenartigen Abschnittes voneinander beabstandet sind. Somit geht der Aufreißabschnitt unmittelbar (ohne Versatz, Stufe) in den laschenartigen Abschnitt über. Dies erleichtert das Entfernen bzw. Aufreißen des Aufreißabschnittes und die Herstellung des Flachzuschnittes der entsprechend ausgebildeten Schutzhülle.

Zudem ist vorstellbar, dass der Verbindeabschnitt stoffeinstückig mit der Basisplatte stoffschlüssig verbunden/ausgebildet ist. In diesem Fall grenzt der Verbindeabschnitt mit dazwischenliegender Klappfalzlinie direkt an die Basisplatte an.

Weiterhin ist es sinnvoll, wenn der Aufreißabschnitt stoffeinstückig mit dem Verbindeabschnitt verbunden ist. Dies erleichtert die Herstellung und Handhabung eines entsprechend ausgebildeten Flachzuschnittes der Schutzhülle.

Zudem kann vorgesehen sein, dass zwei Perforationslinien vorgesehen sind, die nicht-linear (d.h. mit zumindest einer Krümmung / Kurve) ausgebildet sind, in Bezug auf eine die Klappfalzlinie halbierende Linie achsensymmetrisch zueinander angeordnet sind und sich jeweils ausgehend von der Klappfalzlinie zu der der Klappfalzlinie gegenüberliegenden Seitenkante der Klapp- oder Deckplatte (3) durchgängig erstrecken. Diese Variante hat den Vorteil, dass eine derartige Verpackung bzw. Schutzhülle besonders einfach zu fertigen ist. Zudem bringt die nicht-lineare Ausbildung der Perforationslinien, d.h. Perforationslinien ohne spitze Ecken innerhalb des Verlaufs der Perforationslinien, den Vorteil mit sich, dass sich der Aufreißabschnitt besonders effektiv und rückstandslos von dem Verbindeabschnitt trennen lässt.

Zudem kann es vorgesehen sein, dass die Basis-Eingriffseinrichtung der Basisplatte als, insbesondere schlitzförmige, Aufnahme ausgebildet ist, die in unmittelbarer Nähe zu einer der Seitenkanten der Basisplatte angeordnet ist, und die Klapp- oder Deckplatte einen laschenförmigen Abschnitt aufweist, der sich von der Seitenkante der Klapp- oder Deckplatte erstreckt, die im Geschlossenzustand der Seitenkante mit Aufnahme der Basisplatte gegenüberliegt, und der in die Aufnahme der Basisplatte einführbar ist, oder dass die Basis-Eingriffseinrichtung der Basisplatte als laschenförmiger Abschnitt der Basisplatte ausgebildet ist und die Klapp- oder Deckplatte eine im Geschlossenzustand diesen laschenförmigen Abschnitt der Basisplatte aufnehmende Aufnahme aufweist.

Mit anderen Worten ist die Basis-Eingriffseinrichtung als Aufnahme in der Basisplatte oder als laschenartiger Abschnitt der Basisplatte ausgeführt, die bzw. der komplementär zu einer an der Deckplatte vorgesehenen Deckplatten-Eingriffseinrichtung (laschenartiger Abschnitt oder Aufnahme) ausgebildet ist. Auf diese Weise kann der Wirkeingriff zwischen Basisplatte und Deckplatte einfach realisiert werden.

Über den in die Aufnahme eingreifenden laschenartigen Abschnitt der Deckplatte oder Basisplatte können die Seitenkanten der Basisplatte und der Deckplatte einfach und schnell miteinander verbunden werden. Der laschenartige Abschnitt der Deckplatte oder Basisplatte kann außerdem leicht von einem Verwender gegriffen werden.

Vorteilhafterweise ist der gesamte Flachzuschnitt, der die Schutzhülle ausbildet, stoffeinstückig ausgebildet. Das heißt, alle Laschen bzw. der laschenartige Abschnitt der Deckplatte oder Basisplatte, die sich am Flachzuschnitt befinden, sind stoffeinstückig mit diesem (und damit auch aus demselben Material) ausgebildet.

Außerdem ist es hilfreich, wenn der laschenartige Abschnitt der Deckplatte oder Basisplatte durch Materialanhäufung, d.h. durch eine dickere Wandstärke als der Rest der Schutzhülle, vorzugweise, doppelte Wandstärke, stabilisiert ist.

Wenn der laschenartige Abschnitt der Deckplatte oder Basisplatte derartig stabilisiert ist, kann der laschenartige Abschnitt der Deckplatte oder Basisplatte leicht gegriffen werden und es ist sehr unwahrscheinlich, dass der laschenartige Abschnitt der Deckplatte oder Basisplatte einreißt oder sich ablöst.

Vorteilhaft ist es, wenn der laschenartige Abschnitt der Deckplatte oder Basisplatte, von der ihn tragenden Seitenkante (Seitenkante der Deckplatte oder der Basisplatte) nach außen wegragt und, insbesondere an ihrem abgerundeten Ende, eine, insbesondere kreisförmige, runde oder eckige, Aussparung aufweist.

Die Aussparung erleichtert einem Verwender das Greifen des laschenartigen Abschnitts der Deckplatte oder Basisplatte. Indem der laschenartige Abschnitt der Deckplatte oder Basisplatte von der ihn tragenden Seitenkante nach außen wegragt, ist der laschenartige Abschnitt der Deckplatte oder Basisplatte besonders gut zu greifen.

Der laschenförmige Abschnitt der Deckplatte oder Basisplatte kann zungenförmig oder in Form einer Schlinge ausgebildet sein.

Bei der Ausführungsform, bei der die Deckplatte in den Verbindeabschnitt und den Aufreißabschnitt unterteilt ist, ist es besonders vorteilhaft, wenn der laschenförmige Abschnitt stoffeinstückig, d.h. bewegungsfest, mit dem Aufreißabschnitt verbunden ist. Auf diese Weise kann der Aufreißabschnitt besonders leicht an der Lasche gegriffen werden und von dem Verbindeabschnitt (und somit von der mit dem Verbindeabschnitt stoffschlüssig verbundenen Basisplatte) getrennt werden.

Es ist denkbar, dass die Schutzhülle bzw. der sie ausbildende Flachzuschnitt aus einem flexiblen, abriebfesten und sterilisierbaren Material, vorzugsweise aus TPU, Tyvek, Silikon oder Kunststoffen mit vergleichbaren Eigenschaften, hergestellt ist.

Es ist weiterhin vorstellbar, dass das Material, aus dem die Schutzhülle bzw. der sie ausbildende Flachzuschnitt hergestellt ist, eine extrudierte oder geblasene Folie, ein Geflecht, ein Gewebe oder ein Vlies ist (wobei das Material stellenweise tiefgezogene Elemente aufweisen kann). Es kann sich außerdem um ein Monomaterial oder um ein Verbundmaterial handeln.

Wenn die Schutzhülle aus einem derartigen Material ausgebildet ist, ist die Schutzhülle für die Aufnahme von, insbesondere sterilen, Medizinprodukten hervorragend geeignet.

Weiterhin ist vorstellbar, dass die Seitenkante der Basisplatte oder Deckplatte, in deren unmittelbarer Nähe die zumindest abschnittsweise entlang dieser Seitenkante verlaufende Aufnahme aufweist, eine nach außen ragende, insbesondere bogenförmigen, Vorsprung aufweist. Dieser Vorsprung kann von einem Verwender einfach gegriffen werden und vereinfacht die Handhabung der Schutzhülle, insbesondere beim Öffnen der taschenförmigen Schutzhülle.

### Kurzbeschreibung der Figuren

- Fig. 1: ist eine Draufsicht auf einen Flachzuschnitt einer Schutzhülle gemäß einer ersten Ausführungsform;
- Fig. 2A: ist eine Draufsicht auf den Flachzuschnitt aus Fig. 1 mit darauf angeordneten Produkten;
- Fig. 2B: ist eine Draufsicht auf den zur Taschenform geformten Flachzuschnitt aus Fig. 2A;
- Fig. 3A: ist eine bildliche Darstellung der Prozessschritte für das Falten der taschenförmigen Schutzhülle aus dem Flachzuschnitt aus Fig. 1;
- Fig. 3B: ist eine bildliche Darstellung der Prozessschritte für das Entnehmen des Medizinprodukts aus der nach Fig. 3A zusammengebauten Schutzhülle;
- Fig. 4: ist eine Draufsicht auf einen Flachzuschnitt einer Schutzhülle gemäß einer zweiten Ausführungsform;
- Fig. 5A: ist eine Draufsicht auf den Flachzuschnitt aus Fig. 4 mit darauf angeordneten Produkten;
- Fig. 5B: ist eine Draufsicht auf den zur Taschenform geformten Flachzuschnitt aus Fig. 5A;
- Fig. 6: ist eine Draufsicht auf einen Flachzuschnitt einer Schutzhülle gemäß einer dritten Ausführungsform;
- Fig. 7A: ist eine Draufsicht auf den Flachzuschnitt aus Fig. 6 mit darauf angeordnetem Produkt;
- Fig. 7B: ist eine Draufsicht auf den zur Taschenform geformten Flachzuschnitt aus Fig. 7A;
- Fig. 8: ist eine Draufsicht auf einen Flachzuschnitt einer Schutzhülle gemäß einer vierten Ausführungsform;
- Fig. 9A: ist eine Draufsicht auf den Flachzuschnitt aus Fig. 8 mit darauf angeordneten Produkten;
- Fig. 9B: ist eine Draufsicht auf den zur Taschenform geformten Flachzuschnitt aus Fig. 9A;
- Fig. 10: ist eine Draufsicht auf einen Flachzuschnitt einer Schutzhülle gemäß einer fünften Ausführungsform;
- Fig. 11A: ist eine Draufsicht auf den Flachzuschnitt aus Fig. 10 mit darauf angeordneten Produkten;
- Fig. 11B: ist eine Draufsicht auf den zur Taschenform geformten Flachzuschnitt aus Fig. 11A;
- Fig. 12: ist eine Draufsicht auf einen Flachzuschnitt einer Schutzhülle gemäß einer sechsten Ausführungsform;
- Fig. 13A: ist eine Draufsicht auf eine Schutzhülle, die aus dem Flachzuschnitt aus Fig. 12 gefaltet wurde;
- Fig. 13B: ist eine Perspektivansicht der Schutzhülle aus Fig. 13A, in die ein Medizinprodukt eingeführt wird;
- Fig. 13C: ist eine Perspektivansicht auf die Schutzhülle aus Fig. 13B, die zur Taschenform geschlossen ist und das Medizinprodukt einschließt;
- Fig. 14: ist eine bildliche Darstellung der Prozessschritte für das Entnehmen des Medizinprodukts aus der nach Fig. 13A bis Fig. 13C zusammengebauten Schutzhülle;
- Fig. 15A bis Fig. 15 L: zeigen eine erste bis zwölfte Variante der Schutzhülle 1 der sechsten Ausführungsform;
- Fig. 16A: ist eine bildliche Darstellung der Prozessschritte zum Einführen eines Medizinprodukts in eine offenbarungsgemäße Verpackung; und
- Fig. 16B: ist eine bildliche Darstellung der Prozessschritte zum Auspacken eines Medizinprodukts aus der offenbarungsgemäßen Verpackung.

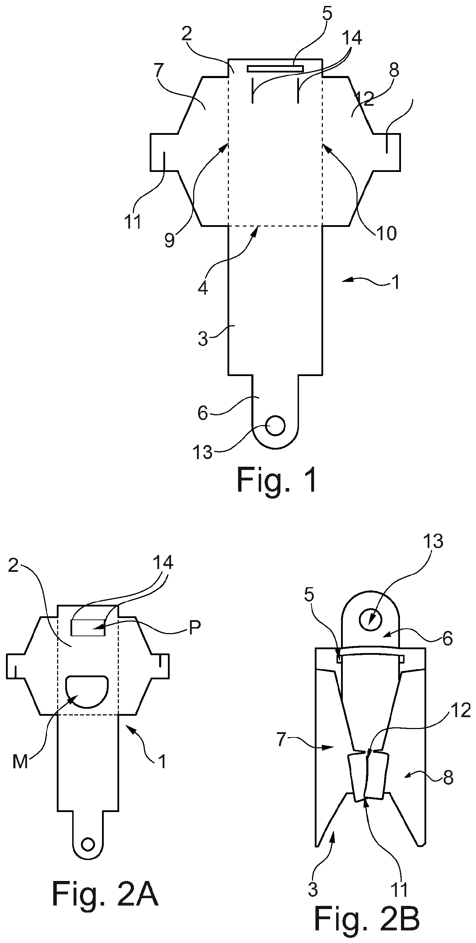

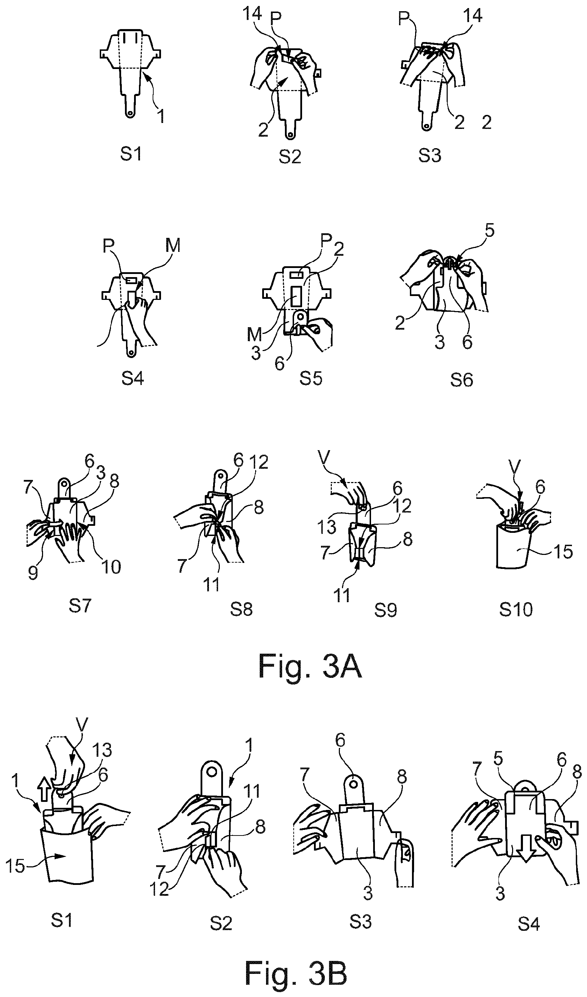

Fig. 1 ist eine Draufsicht auf einen Flachzuschnitt einer Schutzhülle 1 einer ersten Ausführungsform. Der Flachzuschnitt ist aus einem flexiblen Material ausgebildet. Die Schutzhülle 1 dient zum Aufnehmen eines (in Fig. 1 nicht dargestellten) Medizinprodukts, insbesondere eines zu sterilisierenden Medizinprodukts, vorzugweise Implantat mit rauer Oberflächenstruktur. Die Schutzhülle 1 wird aus dem flexiblen Flachzuschnitt geformt, indem der Flachzuschnitt in eine Taschenform geklappt bzw. gefaltet wird (s. Fig. 3A). Die Schutzhülle 1 ist in ihren Abmessungen und in ihrer Formgebung dazu vorgesehen und ausgebildet, in einem das Medizinprodukt aufnehmenden Geschlossenzustand in einer, insbesondere vakuumierbaren, (hier nicht dargestellten) Umverpackung bzw. Sterilverpackung anordenbar zu sein (nachfolgend einfach als Sterilverpackung bezeichnet).

Der Flachzuschnitt der Schutzhülle 1 weist eine im Wesentlichen rechteckförmige Basisplatte 2 mit zwei einander gegenüberliegenden kurzen Seitenkanten und zwei einander gegenüberliegenden langen Seitenkanten auf. An einer kurzen Seitenkante der Basisplatte 2 schließt sich eine Deckplatte 3 mit rechteckförmigem Abschnitt an. Auch der rechteckförmige Abschnitt der Deckplatte 3 weist zwei einander gegenüberliegende kurze Seitenkanten und zwei einander gegenüberliegende lange Seitenkanten auf. Eine Klappfalzlinie 4 entlang der aufeinandertreffenden (und zusammenfallenden) kurzen Seitenkanten von Basisplatte 2 und Deckplatte 3 markiert den Übergang bzw. die Grenze zwischen Basisplatte 2 und Deckplatte 3. Die Deckplatte 3 ist mittels der Klappfalzlinie 4 relativ zur Basisplatte 2 über die Basisplatte 2 faltbar. Die langen Seitenkanten der Basisplatte 2 sind vorteilhafterweise länger als die langen Seitenkanten der Deckplatte 3. An der anderen kurzen Seitenkante der Basisplatte 2, die der Deckplatte 3 abgewandt ist und der Klappfalzlinie 4 (parallel) gegenüberliegt, weist die Basisplatte 2 (innerhalb ihrer Rechteckform) als Basis-Eingriffsvorrichtung eine Aufnahme 5 auf. Die Aufnahme 5 befindet sich dabei direkt unterhalb der entsprechenden kurzen Seitenkante. Die Aufnahme 5 ist zu einem länglichen Schlitz geformt. Die Aufnahme 5 erstreckt sich parallel zur kurzen Seitenkante der Basisplatte 2. Von der kürzeren Seitenkante des rechteckförmigen Abschnitts der Deckplatte 3, die der Basisplatte 2 abgewandt ist, erstreckt sich ein, insbesondere stoffeinstückig damit ausgebildeter, laschenartiger Abschnitt (nachfolgend einfach als "Lasche 6" bezeichnet) der Deckplatte 3 nach außen. Die Deckplatte 3 weist hier also einen rechteckförmigen Abschnitt und einen laschenförmigen Abschnitt auf. Die Lasche 6 und die Aufnahme 5 sind in ihren Abmessungen relativ zueinander so ausgebildet, dass die Lasche 6 in die Aufnahme 5 einführbar ist. Das heißt, die Aufnahme 5 ist mindestens so breit wie die Lasche 6.

An die einander gegenüberliegenden langen Seitenkanten der Basisplatte 2 schließt sich jeweils eine erste Seitenplatte 7 und eine zweite Seitenplatte 8 an. Die jeweils untere Kante der ersten Seitenplatte 7 und der zweiten Seitenplatte 8 verläuft auf Höhe der Klappfalzlinie 4. Insgesamt erstrecken sich die erste und die zweite Seitenplatte 7, 8 nicht über die gesamte Länge der langen Seitenkanten der Basisplatte 2. Die gemeinsame Seitenkante zwischen erster Seitenplatte 7 und Basisplatte 2 ist als Schwenkfalzlinie 9 ausgebildet. Die gemeinsame Seitenkante zwischen zweiter Seitenplatte 8 und Basisplatte 2 ist als Schwenkfalzlinie 10 ausgebildet. Die erste und die zweite Seitenplatte 7, 8 sind mittels der jeweiligen Schwenkfalzlinie 9, 10 relativ zur Basisplatte 2 über die Basisplatte 2 bzw. die Deckplatte 3 faltbar. Die erste und zweite Seitenplatte 7, 8 weisen jeweils an ihren im Geschlossenzustand der Schutzhülle 1 einander überlappenden Überlappungsbereichen einen Einrastschlitz 11, 12 auf. Die Einrastschlitze 11, 12 sind jeweils durch einen Schnitt im Überlappungsbereich der jeweiligen ersten bzw. zweiten Seitenplatte 7, 8 realisiert. Der Einrastschlitz 11 der ersten Seitenplatte 7 (der parallel zur Schwenkfalzlinie 9 verläuft) erstreckt sich von der unteren Kante des Überlappungsbereichs der ersten Seitenplatte 7 bis zu dessen Mitte. Der Einrastschlitz 12 der zweiten Seitenplatte 8 (der parallel zur Schwenkfalzlinie 10 verläuft) erstreckt sich von der oberen Kante des Überlappungsbereichs der zweiten Seitenplatte 8 bis zu dessen Mitte. Natürlich könnten die Einrastschlitze 11, 12 genau andersrum im jeweiligen Überlappungsbereich angeordnet sein. Die Einrastschlitze 11, 12 sind dafür vorgesehen, im Geschlossenzustand formschlüssig ineinanderzugreifen bzw. sich ineinander zu verhaken.

Weiterhin ist zu erkennen, dass die Lasche 6 schmaler als die kurze Seitenkante der Deckplatte 3 ist. Die Lasche 6 ist relativ zu der kurzen Seitenkante mittig positioniert. Die Lasche 6 weist an ihrem abgerundeten freien Ende eine Aussparung 13 auf. Die Aussparung 13 ist kreisförmig. Die Aussparung 13 dient als Griffhilfe. Die Basisplatte 2 weist unterhalb der schlitzartigen Aufnahme 5 zumindest zwei voneinander beabstandete und jeweils zueinander und zu den langen Seitenkanten der Basisplatte 2 parallel verlaufende Schlitze 14 auf. In den Schlitzen 14 kann beispielsweise ein Medizinprodukt oder ein das Medizinprodukt begleitende Packmittel, z.B. ein Trockenmittel, fixiert werden.

Fig. 2A zeigt den aus Fig. 1 bekannten Flachzuschnitt der Schutzhülle 1 mit einem auf der Basisplatte 2 angeordneten Medizinprodukt M. Zwischen den beiden Schlitzen 14 an der Basisplatte 2 ist ein Packmittel P, z.B. ein Trockenmittel, fixiert.

Fig. 2B zeigt die aus dem Flachzuschnitt der Schutzhülle 1 der Fig. 2A gefaltete Schutzhülle 1. Mit anderen Worten ist in Fig. 2B der Geschlossenzustand der Schutzhülle 1 der ersten Ausführungsform gezeigt. Die Deckplatte 3 ist entlang der hier nicht sichtbaren Klappfalzlinie 4 über die Basisplatte 2 gefaltet. Die Lasche 6 ist in die schlitzartige Aufnahme 5 an der Basisplatte 2 eingesteckt. Damit umschließen Basisplatte 2 und Deckplatte 3 das Medizinprodukt M und das Packmittel P. Die erste und die zweite Seitenplatte 7, 8 sind jeweils über die hier nicht sichtbaren Klappfalzlinien 9, 10 über die Deckplatte 3 geklappt. Die Einrastschlitze 11, 12 der Überlappungsbereiche der jeweiligen ersten bzw. zweiten Seitenplatte 7, 8 greifen ineinander und verschließen die Schutzhülle 1. Die Schutzhülle 1 ist auf diese Weise von allen Seiten geschlossen. Das Medizinprodukt M und das Packmittel P verbleiben somit selbst bei Erschütterungen während eines möglichen Transports in der Schutzhülle 1.

Fig. 3A zeigt bildlich die Prozessschritte S1 bis S10 für das Falten der taschenförmigen Schutzhülle 1 aus dem Flachzuschnitt aus Fig. 1. Schritt S1 zeigt den ausgebreiteten Flachzuschnitt der Schutzhülle 1. Im Schritt S2 wird ein Packmittel P mit seiner einen (kurzen) Seite in einen der Schlitze 14 in der Basisplatte 2 eingeschoben. Im Schritt S3 wird die der einen Seite, die im Schritt S2 in den einen Schlitz 14 eingeschoben wurde, gegenüberliegende Seite des Packmittels P in den anderen Schlitz 14 eingeschoben. Somit ist das Packmittel an der Basisplatte 2 fixiert. Im Schritt S4 wird ein Medizinprodukt M auf der Basisplatte 2 positioniert. Im Schritt S5 wird die Deckplatte 3 an der Lasche 6 gegriffen und über die Basisplatte 2 gefaltet. Im Schritt S6 wird die Lasche 6 der Deckplatte 3 in die Aufnahme 5 der Basisplatte 2 eingeführt. Die Schutzhülle 1 ist somit an ihrer Unterseite (mittels der durchgängig ausgebildeten Klappfalzlinie 4) und an ihrer Oberseite (mittels in die Aufnahme 5 eingreifende Lasche 6) geschlossen. Im Schritt S7 wird die erste Seitenplatte 7 entlang der Schwenkfalzlinie 9 über die Deckplatte 3 gefaltet und es wird die zweite Seitenplatte 8 entlang der Schwenkfalzlinie 10 über die Deckplatte 3 gefaltet. Im Schritt S8 werden die Einrastschlitze 11, 12 ineinander verhakt und damit werden die erste und die zweite Seitenplatte 7, 8 fixiert. Die Schutzhülle 1 ist somit auch seitlich geschlossen. Im Schritt S9 hält ein Verwender V die das Medizinprodukt M und das Packmittel P aufnehmende zur Taschenform gefaltete Schutzhülle 1 unter Verwendung der Aussparung 13 als Griffhilfe. Im Schritt S10 packt ein Verwender V unter Verwendung der Aussparung 13 als Griffhilfe die in den Schritten S1 bis S9 vorbereitete Schutzhülle 1 in eine Sterilverpackung 15 ein (s. Pfeilrichtung) und bildet somit eine Ausführungsform der offenbarungsgemäßen Verpackung aus.

Fig. 3B zeigt schematisch Prozessschritte S1 bis S4 für das Entnehmen des Medizinprodukts M aus der nach Fig. 3A gefalteten taschenförmigen Schutzhülle 1. Im Schritt S1 wird die Schutzhülle 1 über die Lasche 6, insbesondere über die Aussparung 13 als Griffhilfe, aus der Sterilverpackung 15 gezogen (s. Pfeilrichtung). Im Schritt S2 werden die ineinander verhakten Einrastschlitze 11, 12 enthakt. Im Schritt S3 werden die erste und die zweite Seitenplatte 7, 8 von der Deckplatte 3 nach außen geklappt und damit aufgefaltet. Im Schritt S4 wird die Lasche 6 aus der Aufnahme 5 gezogen und die Deckplatte 3 von der Basisplatte 2 weggefaltet. Nach Durchführung des Schrittes S4 liegen Medizinprodukt M und Packmittel P offen auf der Basisplatte 2 auf und können von der zum Flachzuschnitt aufgefalteten Schutzhülle 1 entnommen werden (hier nicht dargestellt).

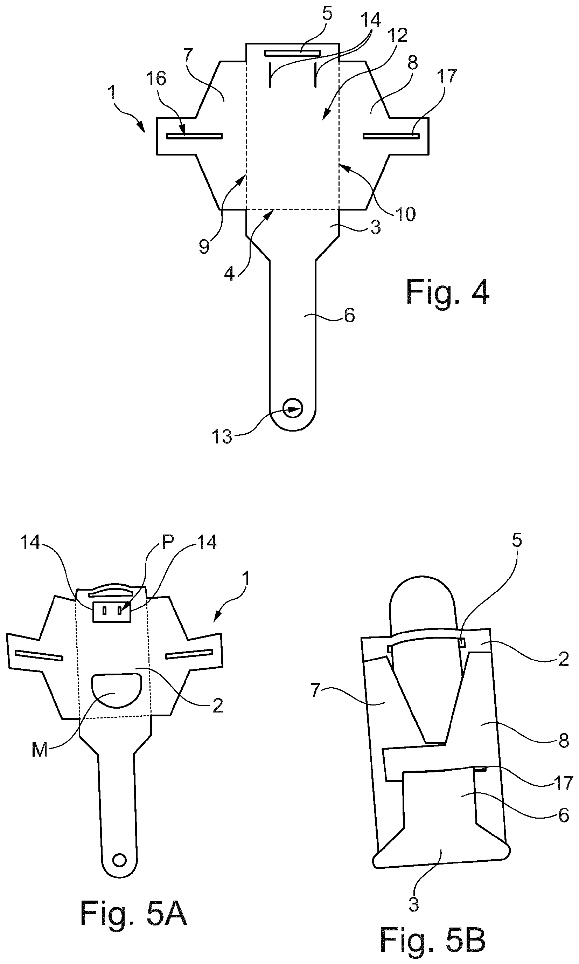

Fig. 4 ist eine Draufsicht auf einen Flachzuschnitt einer Schutzhülle 1 einer zweiten Ausführungsform. Der Flachzuschnitt der Schutzhülle 1 der zweiten Ausführungsform entspricht im Wesentlichen dem Flachzuschnitt der Schutzhülle 1 der ersten Ausführungsform. Die Basisplatte 2 der Schutzhülle 1 der zweiten Ausführungsform ist genauso aufgebaut, wie die Basisplatte 2 der Schutzhülle 1 der ersten Ausführungsform (s. Fig. 1). Auch hier sind an der Basisplatte 2 eine erste und eine zweite Seitenplatte 7, 8 angeordnet, deren Abmessung im Wesentlichen der aus der ersten Ausführungsform gleicht. Im Unterschied zur ersten Ausführungsform weisen die erste und die zweite Seitenplatte 7, 8 in ihren Überlappungsbereichen anstatt der Einrastschlitze 11, 12 jeweils eine schlitzartige Aufnahme 16, 17 auf. Die schlitzartigen Aufnahmen 16, 17 entsprechen in Form und Abmessung jeweils im Wesentlichen der Aufnahme 5 an der Basisplatte 2.

Die Seitenkanten des rechteckförmigen Abschnitts der Deckplatte 3, die die langen Seitenkanten der Basisplatte 2 fortsetzen, sind wesentlich kürzer als die langen Seitenkanten der Basisplatte 2 und sogar wesentlich kürzer als die, d.h. kürzer als die Hälfte der, kurze(n) Seitenkante des rechteckförmigen Abschnitts der Deckplatte 3 bzw. der Basisplatte 2 (bzw. als die Klappfalzlinie 4). Die Lasche 6 der Deckplatte 3 ist dafür wesentlich länger als bei der Schutzhülle 1 der ersten Ausführungsform.

Fig. 5A zeigt eine Draufsicht auf den Flachzuschnitt aus Fig. 4. Hier ist ein Medizinprodukt M auf der Basisplatte 2 angeordnet. Außerdem ist ein Packmittel P in die Schlitze 14 an der Basisplatte 2 eingeführt.

Fig. 5B zeigt die aus dem Flachzuschnitt der Schutzhülle 1 der Fig. 5A gefaltete Schutzhülle 1. Mit anderen Worten ist in Fig. 5B der Geschlossenzustand der taschenförmigen Schutzhülle 1 der zweiten Ausführungsform gezeigt. Die erste Seitenplatte 7 liegt auf der Basisplatte 2 auf. Die zweite Seitenplatte 8 liegt auf der Basisplatte 2 und auf der ersten Seitenplatte 7 auf. Dabei überlappt der Überlappungsbereich der zweiten Seitenplatte 8 den Überlappungsbereich der ersten Seitenplatte 7 von oben. Genauer gesagt liegt die Aufnahme 17 der zweiten Seitenplatte 8 genau über der Aufnahme 16 der ersten Seitenplatte 7. Dabei bilden Aufnahme 16 und Aufnahme 17 der jeweiligen ersten bzw. zweiten Seitenplatte 7, 8 eine durchgängige Aufnahme zur Aufnahme der Lasche 6 aus. Die Deckplatte 3 ist über die erste und die zweite Seitenplatte 7, 8 gefaltet und liegt auf diesen auf. Die Lasche 6 ist in die übereinanderliegenden Aufnahmen 16, 17 der ersten und der zweiten Seitenplatte 7, 8 eingeführt und ist in die Aufnahme 5 der Basisplatte 2 eingeführt. Auch die Schutzhülle 1 der zweiten Ausführungsform ist, wie die der ersten Ausführungsform, in der zusammengefalteten Taschenform von allen Seiten verschlossen.

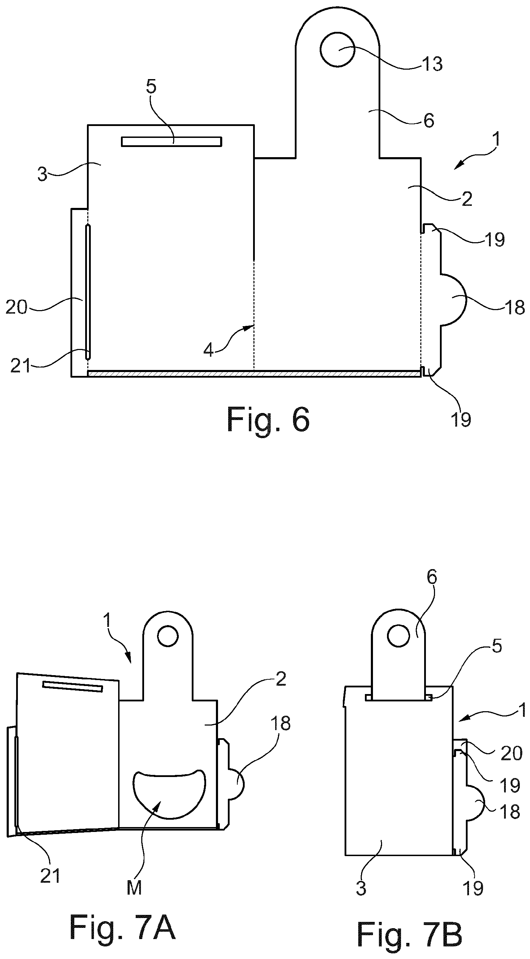

Fig. 6 ist eine Draufsicht auf einen Flachzuschnitt einer Schutzhülle 1 einer dritten Ausführungsform. Hier fallen die langen Seitenkanten der Basisplatte 2 und der Deckplatte 3 zusammen und bilden an dieser Stelle, zumindest abschnittsweise, die Klappfalzlinie 4 aus. Dabei erstreckt sich die Klappfalzlinie 4 nicht über die gesamte Länge der langen Seitenkanten der Basisplatte 2 bzw. der Deckplatte 3. Die Klappfalzlinie 4 erstreckt sich nur von der unteren kurzen Seitenkante der Basisplatte 2 bzw. Deckplatte 3 bis in etwa zur Hälfte der entsprechenden langen Seitenkante. Im übrigen Bereich (die obere Hälfte) der langen Seitenkanten der Basisplatte bzw. der Deckplatte 3 fallen diese nicht zusammen und sind getrennt voneinander ausgebildet (und nicht miteinander verbunden). Im Geschlossenzustand der Schutzhülle 1 ist die Schutzhülle 1 in diesen Bereichen also geöffnet. In dieser Ausführungsform ist im Gegensatz zu den ersten beiden Ausführungsformen die Basisplatte 2 (wie die Deckplatte 2 in den ersten beiden Ausführungsformen) mit einem rechteckförmigen Abschnitt und einem laschenförmigen Abschnitt versehen. An der in der Fig. 4 oberen kurzen Seitenkante des rechteckförmigen Abschnitts weist die Basisplatte 2 den laschenförmigen Abschnitt bzw. die Lasche 6 auf. Auch hier ist die Lasche 6 mit einer kreisförmigen Aussparung 13 versehen. Die Deckplatte 3 weist im Bereich ihrer oberen kurzen Seitenkante die parallel zu dieser Seitenkante verlaufende und von dieser beabstandete Aufnahme 5 auf. Hier ist demnach die lange Seitenkante der Basisplatte 2 geringfügig kürzer als die lange Seitenkante der Deckplatte 3, die die Aufnahme 5 aufweist. Genauer gesagt endet die lange Seitenkante der Basisplatte 2 unterhalb der Aufnahme 5 der Deckplatte 3.

An der der Klappfalzlinie 4 gegenüberliegenden langen Seitenkante weist die Basisplatte 2 eine sich zumindest teilweise über die lange Seitenkante erstreckende Schließlasche 18 auf. Die Schließlasche 18 ist über eine parallel zur Klappfalzlinie 4 verlaufende Schwenkfalzlinie relativ zur Basisplatte 2 schwenkbar. Die Schließlasche 18 weist an ihren beiden in Längsrichtung einander gegenüberliegende Enden jeweils einen Widerhaken 19 auf. An der der Klappfalzlinie 4 gegenüberliegenden langen Seitenkante weist die Deckplatte 3 eine sich zumindest teilweise entlang der langen Seitenkante erstreckende Einstecklasche 20 auf. Die Einstecklasche 20 ist über eine parallel zur Klappfalzlinie 4 verlaufende Schwenkfalzlinie relativ zur Deckplatte 3 schwenkbar. Schließlasche 18 und Einstecklasche 20 sind insbesondere stoffeinstückig mit der Deckplatte 3 bzw. der Basisplatte 2 ausgebildet. Dabei ist zwischen der Deckplatte 3 und der Einstecklasche 20 im Bereich der langen Seitenkante entlang dieser Seitenkante eine schlitzartige Laschenaufnahme 21 ausgebildet. In anderen Worten unterbricht bzw. ersetzt die Laschenaufnahme 21 die lange Seitenkante an dieser Stelle. Die Schließlasche 18 und die Einstecklasche 20 sind auf gleicher Höhe angebracht, sodass im Geschlossenzustand die Schließlasche 18 mit den Widerhaken 19 in die Laschenaufnahme 21 eingreifen kann bzw. in diese eingesteckt werden kann. Die Länge der Schließlasche 18 ist, insbesondere genau, um die Länge der Widerhaken 19 länger als die Laschenaufnahme 21.

Die unteren kurzen Seitenkanten von Basisplatte 2 und Deckplatte 3 werden stoffschlüssig miteinander verbunden, wie durch den schraffierten Bereich dargestellt. Das heißt, im schraffierten Bereich werden Basisplatte 2 und Deckplatte 3 miteinander verklebt, verschweißt oder versiegelt.

Fig. 7A zeigt eine Draufsicht auf den Flachzuschnitt der Schutzhülle 1 aus Fig. 6 mit darauf angeordnetem Medizinprodukt M. Genauer gesagt liegt das Medizinprodukt M auf der Basisplatte 2 auf.

Fig. 7B ist eine Draufsicht auf den zur Schutzhülle 1 geformten Flachzuschnitt aus Fig. 7A. Mit anderen Worten ist in Fig. 7B der Geschlossenzustand der taschenförmigen Schutzhülle 1 der dritten Ausführungsform gezeigt. Die Deckplatte 3 ist über die Basisplatte 2 geklappt und bedeckt somit das Medizinprodukt M. Die Lasche 6 der Basisplatte 2 ist in die Aufnahme 5 an der Deckplatte 3 eingeführt. Die Schließlasche 18 ist in die Laschenaufnahme 21 eingeführt. Die Widerhaken 19 verhindern, dass sich die Schließlasche 18 aus der Laschenaufnahme 21 löst. Mit anderen Worten hintergreifen die Widerhaken 19 die Laschenaufnahme 21. Damit bilden die Schließlasche 18 und die Einstecklasche 20 eine formschlüssige Verbindung aus. Die unteren kurzen Seitenkanten von Basisplatte 2 und Deckplatte 3 sind im hier dargestellten Geschlossenzustand miteinander stoffschlüssig verbunden (verschweißt, verklebt oder versiegelt).

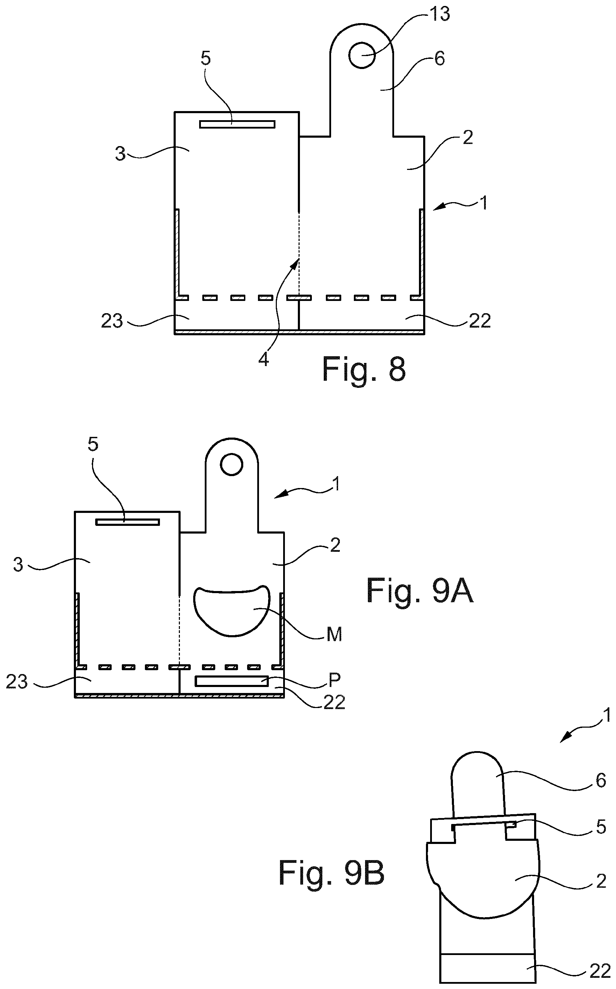

Fig. 8 eine Draufsicht auf einen Flachzuschnitt einer Schutzhülle 1 einer vierten Ausführungsform. Die Schutzhülle 1 der vierten Ausführungsform entspricht im Wesentlichen der der dritten Ausführungsform. Allerdings entfallen bei der Schutzhülle der vierten Ausführungsform nach Fig. 8 die Schließlasche 18 und auch die Einstecklasche 20. Anstatt dessen weisen die langen Seitenkanten von Basisplatte 2 und Deckplatte 3 in dem Bereich, der den kurzen Seitenkanten, die mit Aufnahme 5 bzw. Lasche 6 versehen sind, abgewandt sind, Bereiche zur stoffschlüssigen Verbindung auf (s. schraffierte Bereiche). Die schraffierten Bereiche entlang der kurzen Seitenkanten sind unterbrochen. Das heißt, es werden voneinander separierte Punkte bzw. Bereiche der unteren kurzen Seitenkanten stoffschlüssig miteinander verbunden.

Weiterhin grenzt an die untere Seitenkante der Basisplatte 2 eine, insbesondere stoffeinstückig damit ausgebildete, erste Zusatzlasche 22 an und an die untere Seitenkante der Deckplatte 3 grenzt eine, insbesondere stoffeinstückig damit ausgebildete, erste Zusatzlasche 23 an. Die erste Zusatzlasche 22 und die erste Zusatzlasche 23 sind an ihrer der kurzen Seitenkante der Basisplatte 2 bzw. Deckplatte 3 abgewandten bzw. gegenüberliegenden (unteren) Seitenkante durchgängig mit Bereichen zur stoffschlüssigen Verbindung versehen (s. schraffierter Bereich). Im Geschlossenzustand liegt die erste Zusatzlasche 23 auf der ersten Zusatzlasche 22 auf und ist an den Stellen der schraffierten Bereiche mit dieser stoffschlüssig verbunden. Damit bildet die erste Zusatzlasche 23 in Kooperation mit der ersten Zusatzlasche 22 ein Zusatzfach aus, das von dem von der Basisplatte 2 und der Deckplatte 3 definierten Hauptfach räumlich getrennt ist. In dem Zusatzfach kann also ein weiteres Medizinprodukt oder Packmittel getrennt von einem Medizinprodukt und ggf. Packmittel im Hauptfach angeordnet werden.

Fig. 9A zeigt eine Draufsicht auf den Flachzuschnitt der Schutzhülle 1 aus Fig. 8. Dabei ist ein Medizinprodukt M auf der Basisplatte 2 angeordnet. Dieses ist dann im Geschlossenzustand im Hauptfach angeordnet. Außerdem ist ein Packmittel P auf der ersten Zusatzlasche 22, die an die Basisplatte 2 angrenzt, angeordnet. Dieses ist dann im Geschlossenzustand im Zusatzfach angeordnet.

Fig. 9B ist eine Draufsicht auf den zur Schutzhülle 1 geformten Flachzuschnitt aus Fig. 9A. Mit anderen Worten ist in Fig. 9B der Geschlossenzustand der Schutzhülle 1 der vierten Ausführungsform gezeigt. In dieser Darstellung ist die Basisplatte 2 dem Betrachter zugewandt. Deckplatte 3 und Basisplatte 2 bilden zusammen das Hauptfach aus, in dem das Medizinprodukt M angeordnet ist. Die Seitenkanten von Deckplatte 3 und Basisplatte 2 sind in den schraffierten Bereichen, die in Fig. 8 dargestellt sind, miteinander stoffschlüssig verbunden. Die Lasche 6 der Basisplatte 2 ist in die damit korrespondierende Aufnahme 5 der Deckplatte 3 eingeführt. Die erste Zusatzlasche 23 bildet zusammen mit der ersten Zusatzlasche 22 das Zusatzfach aus, in dem das Packmittel P angeordnet ist. Die unteren Seitenkanten der ersten Zusatzlaschen 22, 23 sind im schraffierten Bereich (vgl. Fig. 8) miteinander stoffschlüssig verbunden. Die stoffschlüssige Verbindung im Bereich zwischen Basisplatte 2 bzw. Deckplatte 3 sowie den ersten Zusatzlaschen 22, 23 trennt dabei Hauptfach und Zusatzfach räumlich voneinander ab. Das Hauptfach ist an allen Seiten verschlossen. Das Zusatzfach ist an seinen breiten (kurzen) Seitenkanten geöffnet.

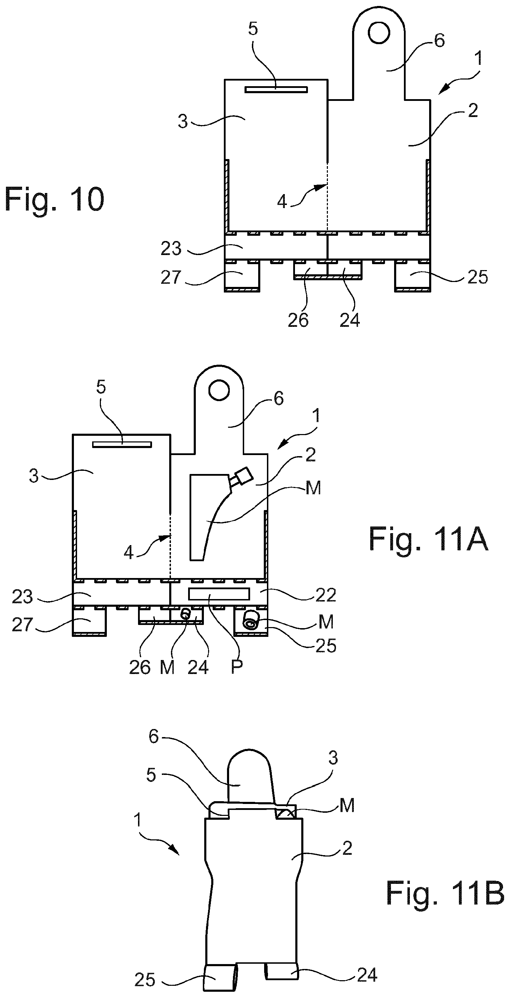

Fig. 10 ist eine Draufsicht auf einen Flachzuschnitt einer Schutzhülle 1 einer fünften Ausführungsform. Die Schutzhülle 1 der fünften Ausführungsform entspricht im Wesentlichen der Schutzhülle 1 der vierten Ausführungsform. Im Unterschied zur Schutzhülle 1 der vierten Ausführungsform weist die der fünften Ausführungsform unterhalb der ersten Zusatzlasche 22 der Basisplatte 2 zwei voneinander beabstandete zweite Zusatzlaschen 24, 25 auf. Die zweite Zusatzlasche 24 ist kleiner als die zweite Zusatzlasche 25. Die äußere Seitenkante der zweiten Zusatzlasche 24 setzt die Klappfalzlinie 4 fort. Die äußere Seitenkante der zweiten Zusatzlasche 25 setzt die der Klappfalzlinie 4 gegenüberliegende lange Seitenkante der Basisplatte 2 fort. Die inneren Seitenkanten der zweiten Zusatzlasche 24 und der zweiten Zusatzlasche 25 sind einander zugewandt. Spiegelbildlich dazu mit der Klappfalzlinie 4 als Mittellinie befinden sich zwei voneinander beabstandete zweite Zusatzlaschen 26, 27 unterhalb der ersten Zusatzlasche 23 der Deckplatte 3. Die zweite Zusatzlasche 26 ist kleiner als die zweite Zusatzlasche 27. Die äußere Seitenkante der zweiten Zusatzlasche 26 setzt die Klappfalzlinie 4 fort und fällt somit mit der äußeren Seitenkante der zweiten Zusatzlasche 24 zusammen. Die äußere Seitenkante der zweiten Zusatzlasche 27 setzt die der Klappfalzlinie 4 gegenüberliegende lange Seitenkante der Deckplatte 3 fort. Die inneren Seitenkanten der zweiten Zusatzlasche 26 und der zweiten Zusatzlasche 27 sind einander zugewandt.

Im Geschlossenzustand kooperiert die zweite Zusatzlasche 24 mit der zweiten Zusatzlasche 26, sodass die zweite Zusatzlasche 26 auf der zweiten Zusatzlasche 24 (flächendeckend) aufliegt und ein Zusatzfach ausbildet. Daher sind die zweite Zusatzlasche 24 und die zweite Zusatzlasche 26 gleich groß. Im Geschlossenzustand kooperiert außerdem die zweite Zusatzlasche 25 mit der zweiten Zusatzlasche 27, sodass die zweite Zusatzlasche 27 auf der zweiten Zusatzlasche 25 (flächendeckend) aufliegt und ein weiteres Zusatzfach ausbildet. Daher sind die zweite Zusatzlasche 25 und die zweite Zusatzlasche 27 gleich groß. Zum Ausbilden der Zusatzfächer sind die jeweils unteren Seitenkanten der zweiten Zusatzlaschen 24 bis 27 durchgängig mit einem Bereich zur stoffschlüssigen Verbindung (s. schraffierter Bereich) mit der Seitenkante der jeweils korrespondierenden zweiten Zusatzlasche 24 bis 27 versehen.

Im Unterschied zu der Schutzhülle 1 der vierten Ausführungsform ist der Bereich der stoffschlüssigen Verbindung im Bereich der unteren Seitenkanten der ersten Zusatzlaschen 22, 23 nicht durchgängig, sondern unterbrochen ausgebildet. Insgesamt weist die zur Taschenform gefaltete Schutzhülle 1 dann also drei Zusatzfächer auf. Alle drei Zusatzfächer sind räumlich voneinander und vom Hauptfach getrennt.

Fig. 11 A ist eine Draufsicht auf den Flachzuschnitt der Schutzhülle 1 aus Fig. 10 mit darauf angeordneten Produkten. Genauer gesagt ist ein Medizinprodukt M auf der Basisplatte 2 angeordnet. Auf der ersten Zusatzlasche 22, die an die Basisplatte 2 angrenzt, ist ein Packmittel P angeordnet. Auf den zweiten Zusatzlaschen 24, 25, die jeweils an die erste Zusatzlasche 22 angrenzen, ist jeweils ein Medizinprodukt M angeordnet.

Fig. 11B ist eine Draufsicht auf den zur Schutzhülle 1 geformten Flachzuschnitt aus Fig. 11A. Mit anderen Worten ist in Fig. 11B der Geschlossenzustand der taschenförmigen Schutzhülle 1 der fünften Ausführungsform gezeigt. In dieser Darstellung ist dem Betrachter die Basisplatte 2 zugewandt. Basisplatte 2 und Deckplatte 2 bilden im Geschlossenzustand das Hauptfach aus, in dem das Medizinprodukt M angeordnet ist. Das Packmittel P ist im durch die erste Zusatzlasche 22 in Kooperation mit der ersten Zusatzlasche 23 ausgebildeten Zusatzfach angeordnet. Jeweils ein weiteres Medizinprodukt M ist in dem durch die zweite Zusatzlasche 24 in Kooperation mit der zweiten Zusatzlasche 26 ausgebildeten Zusatzfach und in dem durch die zweite Zusatzlasche 25 in Kooperation mit der zweiten Zusatzlasche 27 ausgebildeten Zusatzfach angeordnet.

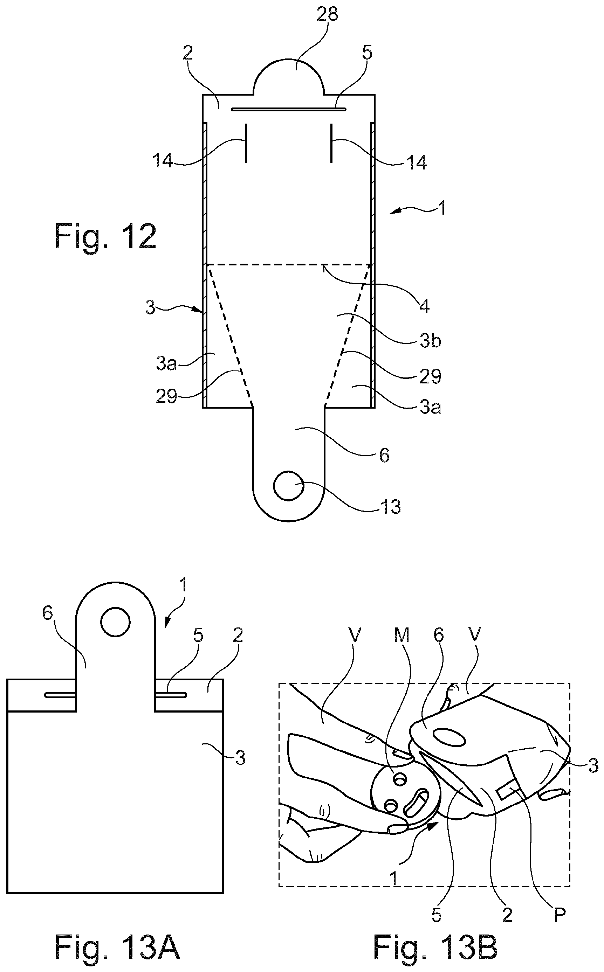

Fig. 12 ist eine Draufsicht auf einen Flachzuschnitt einer Schutzhülle 1 einer sechsten Ausführungsform. Der Flachzuschnitt der Schutzhülle 1 der sechsten Ausführungsform ähnelt in seinem Grundaufbau dem Flachzuschnitt der Schutzhülle 1 gemäß der ersten Ausführungsform. Es wird nachfolgend insbesondere auf die Unterschiede des Flachzuschnitts der Schutzhülle 1 der sechsten Ausführungsform zu dem Flachzuschnitt der Schutzhülle 1 der ersten Ausführungsform eingegangen.

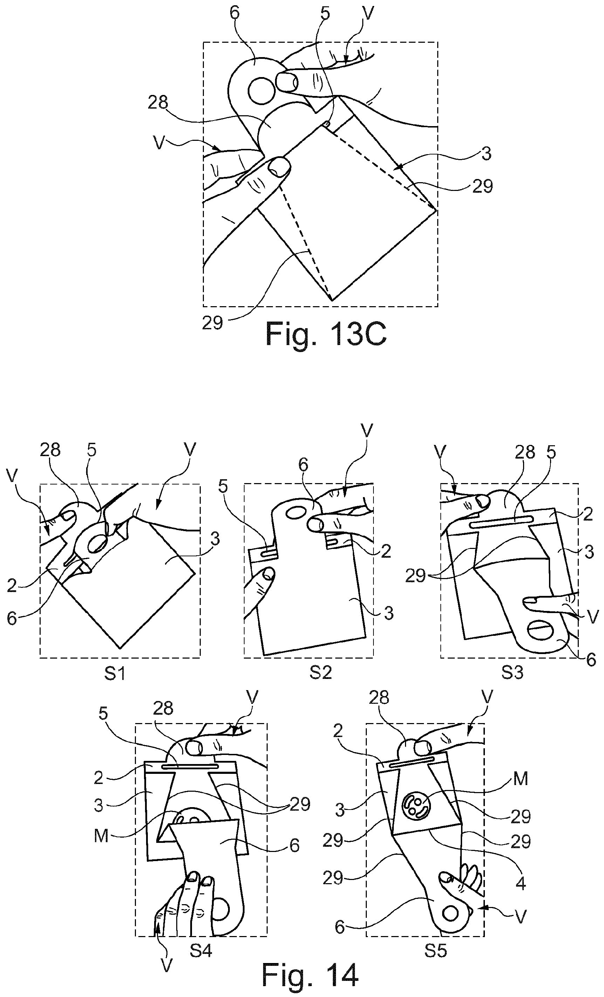

Auch hier ist die kurze gemeinsame Seitenkante von Basisplatte 2 und Deckplatte 3 als Klappfalzlinie 4 ausgebildet. Von der anderen kurzen Seitenkante, in deren unmittelbarer Nähe die Aufnahme 5 angeordnet ist, erstreckt sich ein, insbesondere bogenförmiger, Vorsprung 28 nach außen. Dieser kann insbesondere beim Entnehmen eines Medizinprodukts M**,** hier nicht dargestellt, aus dem zur taschenförmigen Schutzhülle 1 gefalteten Flachzuschnitt besonders einfach von einem Verwender V gegriffen werden und erleichtert somit das Öffnen der taschenförmigen Schutzhülle 1 (s. Fig. 14).

Weiterhin ist die Deckplatte 3 in zwei Abschnitte untergliedert: einen Verbindeabschnitt 3a und einen Aufreißabschnitt 3b. Der Verbindeabschnitt 3a wird vom Aufreißabschnitt 3b in zwei Hälften unterteilt. Verbindeabschnitt 3a und Aufreißabschnitt 3b sind über zwei Perforationslinien 29 voneinander abgegrenzt. Verbindeabschnitt 3a und Aufreißabschnitt 3b sind stoffeinstückig miteinander ausgebildet, wobei die Perforationslinien 29 eine Sollbruchstelle zwischen den beiden Abschnitten 3a, 3b definieren. Die Perforationslinien 29 erstrecken sich von den beiden einander gegenüberliegenden Enden der Klappfalzlinie 4 aus und laufen konisch bzw. schräg in Richtung der der Klappfalzlinie 4 gegenüberliegenden kurzen Seitenkante der Deckplatte 3 aufeinander zu. Genauer gesagt sind die Perforationslinien 29 hier linear ausgebildet. Die Perforationslinien 29 sind achsensymmetrisch (in Bezug auf eine die kurzen Seitenkanten der Deckplatte 3 halbierende Linie) zueinander angeordnet bzw. ausgebildet. Die Perforationslinien 29 enden an der / münden in die der Klappfalzlinie 4 gegenüberliegende(n) kurzen Seitenkante der Deckplatte 3 und sind an dieser Stelle um die Breite der Lasche 6 voneinander beabstandet. Die Lasche 6 der Deckplatte 3 ist stoffeinstückig mit dem Aufreißabschnitt 3b verbunden. Die Lasche 6 ragt ausgehend von der der Klappfalzlinie 4 gegenüberliegenden kurzen Seitenkante der Deckplatte 3 von der Deckplatte 3 weg.

Die beiden Hälften des Verbindeabschnitts 3a bilden jeweils die langen Seitenkanten der Deckplatte 3 aus. Die langen Seitenkanten der Deckplatte 3 verlaufen senkrecht zur Klappfalzlinie 4 und grenzen an diese an. Die langen Seitenkanten der Deckplatte 3 weisen über ihre gesamte Länge einen Bereich auf, über den die Deckplatte stoffschlüssig mit der Basisplatte 2 verbindbar ist, wie durch den schraffierten Bereich dargestellt. Die Basisplatte 2 weist an ihren langen Seitenkanten, die senkrecht zur Klappfalzlinie 4 sind und an diese angrenzen, ebenfalls einen schraffierten Bereich auf, der zur stoffschlüssigen Verbindung mit dem entsprechenden Bereich der Deckplatte 3 vorgesehen ist. Der schraffierte Bereich erstreckt sich ausgehend von der Klappfalzlinie 4 in Richtung der der Klappfalzlinie 4 gegenüberliegenden kurzen Seitenkante der Basisplatte 2. Der schraffierte Bereich der Basisplatte 2 ist genauso lang wie der schraffierte Bereich der Deckplatte 3. Der senkrechte Abstand zwischen den zwei kurzen Seitenkanten der Basisplatte ist größer als der senkrechte Abstand zwischen den zwei kurzen Seitenkanten der Deckplatte 3.

Der Flachzuschnitt der Schutzhülle 1 weist auch in dieser Ausführungsform in der Basisplatte 2 die parallel zueinander verlaufenden und senkrecht zur Klappfalzlinie 4 ausgerichteten Schlitze 14 zur Aufnahme eines Medizinprodukts oder eines das Medizinprodukt begleitenden Packmittels, z.B. eines Trockenmittels.

Vorteilhafterweise ist für den Flachzuschnitt der Schutzhülle 1 gemäß der sechsten Ausführungsform im Vergleich mit den Schutzhüllen 1 der ersten bis fünften Ausführungsform am wenigsten Material erforderlich, da der Flachzuschnitt der Schutzhülle 1 gemäß der sechsten Ausführungsform keine Seitenlaschen oder Zusatzlaschen aufweist.

Fig. 13A zeigt eine Draufsicht auf eine Schutzhülle 1, die aus dem Flachzuschnitt der Schutzhülle 1 gemäß der sechsten Ausführungsform (vgl. Fig. 12) gefaltet wurde. Die Deckplatte 3 ist über die Basisplatte 2 geklappt und die Deckplatte 3 und die Basisplatte 2 sind im jeweils schraffierten Bereich (hier nicht dargestellt, s. Fig. 12) stoffschlüssig miteinander verbunden. Damit bildet die in Fig. 13A gezeigte Schutzhülle 1 eine einseitig geöffnete Tasche aus.

Fig. 13B ist eine Perspektivansicht der Schutzhülle 1 aus Fig. 13A, in die ein Medizinprodukt M (von einem Verwender V) eingeführt wird. Das Medizinprodukt M wird dabei im Gegensatz zu den Schutzhüllen 1 der ersten bis fünften Ausführungsform erst dann in die Schutzhülle 1 gemäß der sechsten Ausführungsform eingeführt, nachdem die Seitenkanten der Basisplatte 2 und der Deckplatte 3 verschlossen wurden. In den Schlitzen 14 ist ein Packmittel P, bspw. ein Trockenmittel, fixiert.

Fig. 13C ist eine Perspektivansicht auf die Schutzhülle 1 aus Fig. 13B, die zur Taschenform geschlossen ist und das Medizinprodukt M (sowie das Packmittel P) einschließt. Die Schutzhülle 1 ist im Bereich der der Klappfalzlinie 4 gegenüberliegenden kurzen Seitenkanten der Basisplatte 2 und der Deckplatte 3 verschlossen. Dazu ist die Lasche 6 in die Aufnahme 5 eingeführt. Die in Fig. 13C dargestellte Schutzhülle 1 ist somit nach allen Seiten hin verschlossen.

Fig. 14 ist eine bildliche Darstellung der Prozessschritte S1 bis S5 für das Entnehmen des Medizinprodukts M aus der nach Fig. 13A bis Fig. 13C zusammengebauten Schutzhülle 1. Im Schritt S1 ist dargestellt, wie die Lasche 6 von einem Verwender V aus der Aufnahme 5 herausgezogen / entfernt wird. Im Schritt S2 greift der Verwender V mit einer Hand die Lasche 6. Im Schritt S3 greift der Verwender V mit einer anderen Hand der Vorsprung 28 zum Fixieren der Basisplatte 2. Mit der einen Hand bringt der Verwender V eine Zugkraft auf die Lasche 6 auf, sodass sich der Aufreißabschnitt 3b von dem Verbindeabschnitt 3a entlang der Perforationslinie 29 löst. Der Verbindeabschnitt 3a ist und bleibt stoffschlüssig mit der Basisplatte 2 verbunden. In Schritt S4 ist der Aufreißabschnitt 3b im Vergleich zu Schritt S3 bereits weiter von dem Verbindeabschnitt 3b getrennt, sodass das Medizinprodukt M zumindest teilweise von dem Aufreißabschnitt 3b freigelegt ist. Im Schritt S5 sind Aufreißabschnitt 3b und Verbindeabschnitt 3a vollständig voneinander getrennt. Das Medizinprodukt M liegt vollständig frei und kann von dem Verwender V aus der Schutzhülle 1 entnommen werden.

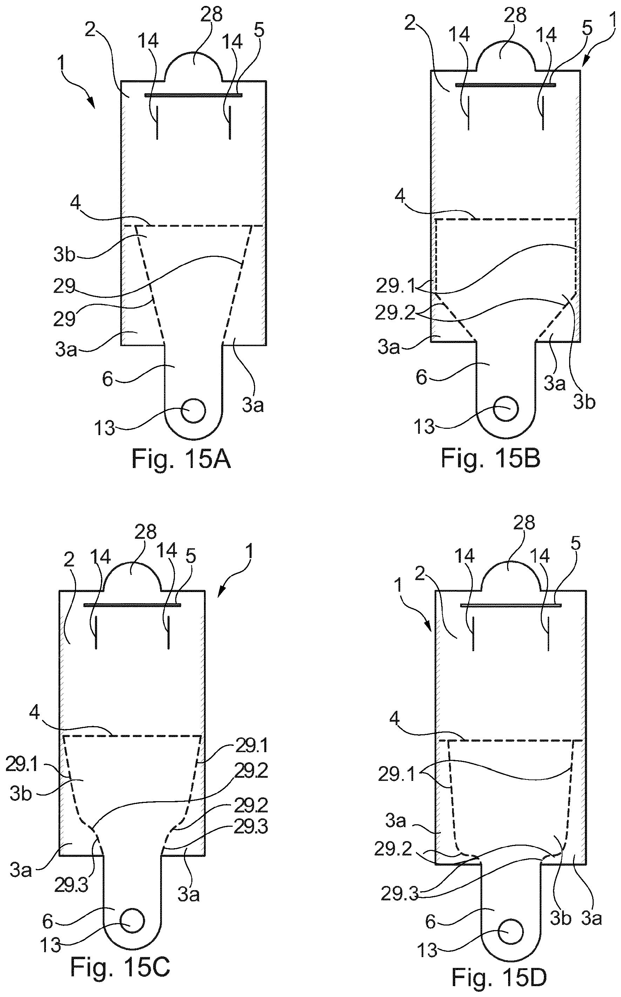

Fig. 15A ist eine Draufsicht auf einen Flachzuschnitt einer Schutzhülle 1 einer ersten Variante der Schutzhülle 1 nach der sechsten Ausführungsform. Der Flachzuschnitt der Schutzhülle 1 der ersten Variante ist bis auf den nachfolgend beschriebenen Unterschied identisch mit der Schutzhülle 1 der sechsten Ausführungsform.

Bei der Schutzhülle 1 der ersten Variante enden die beiden Perforationslinien 29 ebenfalls an der Klappfalzlinie 4, jedoch nicht an deren einander gegenüberliegenden Enden, sondern jeweils beabstandet zu den einander gegenüberliegenden Enden der Klappfalzlinie 4. Somit ist bei der Schutzhülle 1 der ersten Variante die Fläche des zwischen den Perforationslinien 29 definierten Aufreißabschnittes 3b geringfügig kleiner als die Fläche des Aufreißabschnittes 3b der Schutzhülle 1 nach der sechsten Ausführungsform.

Fig. 15B ist eine Draufsicht auf einen Flachzuschnitt einer Schutzhülle 1 einer zweiten Variante der Schutzhülle 1 nach der sechsten Ausführungsform. Der Flachzuschnitt der Schutzhülle 1 der zweiten Variante ist bis auf den nachfolgend beschriebenen Unterschied identisch mit der Schutzhülle 1 der sechsten Ausführungsform.

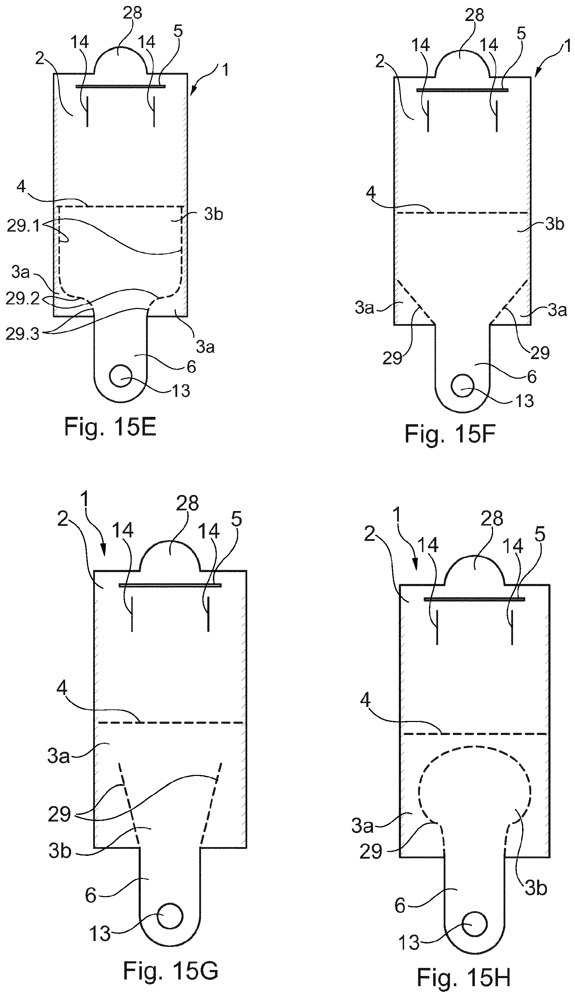

Bei der Schutzhülle 1 der zweiten Variante erstrecken sich jeweilige erste (linear ausgebildete) Perforationslinienabschnitte 29.1 der beiden Perforationslinien 29 ausgehend von den einander gegenüberliegenden Enden der Klappfalzlinie 4 zunächst im Wesentlichen parallel zu bzw. entlang der langen Seitenkanten der Deckplatte 3 (und zwar bis über die Hälfte der Länge der Deckplatte 3). An dieser Stelle enden die ersten Perforationslinienabschnitte 29.1 und an diese schließen sich unmittelbar / direkt zweite (linear ausgebildete) Perforationslinienabschnitte 29.2 an, die sich ausgehend von dem Verbindungspunkt zwischen ersten und zweiten Perforationslinienabschnitten 29,1, 29.2 schräg in Richtung und bis zu der der Klappfalzlinie 4 gegenüberliegenden kurzen Seitenkante der Deckplatte 3 erstrecken. Die zweiten Perforationslinienabschnitte 29.2 sind an dieser Stelle um die Breite der Lasche 6 voneinander beabstandet. Im Fall der zweiten (und der später beschriebenen sowie in Fig. 15E dargestellten fünften) Variante der Schutzhülle 1 schließen die ersten Perforationslinienabschnitte 29.1 unmittelbar an die (schraffiert dargestellten) die langen Seitenkanten der Deckplatte 3 definierenden Bereiche der Deckplatte 3 an, die für eine stoffschlüssige Verbindung mit der Basisplatte 2 ausgebildet sind. Mit anderen Worten liegen die ersten Perforationslinienabschnitte 29.1 unmittelbar neben bzw. benachbart zu den Bereichen der Deckplatte 3 an, die für eine stoffschlüssige Verbindung mit der Basisplatte 2 ausgebildet sind.

Fig. 15C ist eine Draufsicht auf einen Flachzuschnitt einer Schutzhülle 1 einer dritten Variante der Schutzhülle 1 nach der sechsten Ausführungsform. Der Flachzuschnitt der Schutzhülle 1 der dritten Variante ist bis auf den nachfolgend beschriebenen Unterschied identisch mit der Schutzhülle 1 der sechsten Ausführungsform.

Bei der Schutzhülle 1 der dritten Variante sind die beiden Perforationslinien 29 nicht linear ausgebildet. Ausgehend von den beiden einander gegenüberliegenden Enden der Klappfalzlinie 4 erstrecken sich die beiden ersten Perforationslinienabschnitte 29.1 in Richtung der der Klappfalzlinie gegenüberliegenden kurzen Seitenkante der Deckplatte 3 und laufen dabei konisch bzw. schräg aufeinander zu. Dabei erstrecken sich die ersten Perforationslinienabschnitte 29.1 bis etwas über die Hälfte der Länge der Deckplatte 3. An die ersten Perforationslinienabschnitte 29.1 schließen unmittelbar zweite Perforationslinienabschnitte 29.2 an, die sich mit einer deutlich flacheren Steigung als die ersten Perforationslinienabschnitte 29.1 ebenfalls schräg nach innen und somit zueinander erstrecken. Der Übergang zwischen ersten und zweiten Perforationslinienabschnitten 29.1 und 29.2 ist als abgerundete Ecke (nichtlinear!) ausgebildet. An die zweiten Perforationslinienabschnitte 29.2 schließen sich unmittelbar dritte Perforationslinienabschnitte 29.3 an, die sich mit einer steileren Steigung als die zweiten Perforationslinienabschnitte 29.2 schräg nach innen und somit zueinander erstrecken. Der Übergang zwischen zweiten und dritten Perforationslinienabschnitten 29.2 und 29.3 ist als abgerundete Ecke ausgebildet. Die dritten Perforationslinienabschnitte 29.3 enden an der der Klappfalzlinie 4 gegenüberliegenden kurzen Seitenkante der Deckplatte 3. Die dritten Perforationslinienabschnitte 29.3 sind an dieser Stelle um die Breite der Lasche 6 voneinander beabstandet. Vorzugsweise entspricht die Steigung der dritten Perforationslinienabschnitte 29.3 der Steigung der ersten Perforationslinienabschnitte 29.1.

Fig. 15D ist eine Draufsicht auf einen Flachzuschnitt einer Schutzhülle 1 einer vierten Variante der Schutzhülle 1 nach der sechsten Ausführungsform. Der Flachzuschnitt der Schutzhülle 1 der vierten Variante ist bis auf den nachfolgend beschriebenen Unterschied identisch mit der Schutzhülle 1 der dritten Variante.

Hier sind die Perforationslinien insgesamt nicht-linear (mit Krümmungen / Kurven) ausgebildet. Bei der Schutzhülle 1 der vierten Variante erstrecken sich die ersten Perforationslinienabschnitte 29.1 jeweils von einer auf der Klappfalzlinie 4 liegenden Stelle, die von den einander gegenüberliegenden Ende der Klappfalzlinie 4 (geringfügig, bspw. einige mm bis maximal 2cm, bevorzugt maximal 1cm) beabstandet ist. Die ersten Perforationslinienabschnitte 29.1 verlaufen schräg in Richtung zu der der Klappfalzlinie 4 gegenüberliegenden kurzen Seitenkante der Deckplatte 3 und laufen somit konisch aufeinander zu. Die Steigung der ersten Perforationslinienabschnitte 29.1 der vierten Variante ist stärker als die Steigung der ersten Perforationslinienabschnitte 29.1 der dritten Variante. Hier erstrecken sich die ersten Perforationslinienabschnitte 29.1 bis über ca. ¾ der Länge der Deckplatte 3. Die zweiten Perforationslinienabschnitte 29.2 der vierten Variante haben eine geringere Steigung als die der dritten Variante und verlaufen nahezu parallel zur Klappfalzlinie 4. Die dritten Perforationslinienabschnitte 29.3 der vierten Variante sind deutlich kürzer (nur einige mm bis max. 2cm, bevorzugt max. 1cm, lang) als die der dritten Variante. An der Stelle, an der die beiden Perforationslinien 29 in die der Klappfalzlinie 4 gegenüberliegende kurze Seitenkante der Deckplatte 3 münden, sind sie um die Breite der Lasche 6 voneinander beabstandet.

Fig. 15E ist eine Draufsicht auf einen Flachzuschnitt einer Schutzhülle 1 einer fünften Variante der Schutzhülle 1 nach der sechsten Ausführungsform. Der Flachzuschnitt der Schutzhülle 1 der fünften Variante ist bis auf den nachfolgend beschriebenen Unterschied identisch mit der Schutzhülle 1 der dritten Variante.

Hier sind die Perforationslinien nicht-linear (mit Krümmungen / Kurven) ausgebildet. Bei der Schutzhülle 1 der fünften Variante erstrecken sich die ersten Perforationslinienabschnitte 29.1 ausgehend von den einander gegenüberliegenden Enden der Klappfalzlinie 4 parallel zu bzw. entlang der langen Seitenkanten der Deckplatte 3. Die zweiten Perforationslinienabschnitte 29.2 der fünften Variante haben eine geringere Steigung als die der dritten Variante und verlaufen nahezu parallel zur Klappfalzlinie 4. Die dritten Perforationslinienabschnitte 29.3 der fünften Variante sind geringfügig kürzer als die der dritten Variante.

Fig. 15F ist eine Draufsicht auf einen Flachzuschnitt einer Schutzhülle 1 einer sechsten Variante der Schutzhülle 1 nach der sechsten Ausführungsform. Der Flachzuschnitt der Schutzhülle 1 der sechsten Variante ist bis auf den nachfolgend beschriebenen Unterschied identisch mit der Schutzhülle 1 der sechsten Ausführungsform.

Im Gegensatz zu der Schutzhülle 1 der sechsten Ausführungsform und im Gegensatz zu den Schutzhüllen 1 der ersten bis fünften Variante erstrecken sich die beiden (linearen) Perforationslinien 29 der sechsten Variante nicht ausgehend von der Klappfalzlinie 4 zu der der Klappfalzlinie 4 gegenüberliegenden kurzen Seitenkante der Deckplatte 4. Die beiden Perforationslinien 29 der sechsten Variante erstrecken sich von (einer Stelle, die ausgehend von der Klappfalzlinie 4 oberhalb der Hälfte der Länge der Deckplatte 3 liegt, an) den langen Seitenkanten der Deckplatte 3 schräg zu der der Klappfalzlinie 4 gegenüberliegenden kurzen Seitenkante der Deckplatte 4. In anderen Worten schneidet jede Perforationslinie 29 jeweils eine der langen Seitenkanten der Deckplatte 3 und die der Klappfalzlinie 4 gegenüberliegende kurze Seitenkante der Deckplatte 3.

Fig. 15G ist eine Draufsicht auf einen Flachzuschnitt einer Schutzhülle 1 einer siebten Variante der Schutzhülle 1 nach der sechsten Ausführungsform. Der Flachzuschnitt der Schutzhülle 1 der siebten Variante ist bis auf den nachfolgend beschriebenen Unterschied identisch mit der Schutzhülle 1 der ersten Variante.

Bei der Schutzhülle 1 schließen die (linearen) Perforationslinien 29 nicht an die Klappfalzlinie 4 an, sondern erstrecken sich von einer ersten Stelle innerhalb der Deckplatte 3, die einen (Längs-)Abstand zu der Klappfalzlinie 4 hat, (schräg) zu der der Klappfalzlinie 4 gegenüberliegenden kurzen Seitenkante der Deckplatte 3. Die erste Stelle ist um weniger als die Hälfte der Länge der Deckplatte 3 von der Klappfalzlinie 4 beabstandet.

Fig. 15H ist eine Draufsicht auf einen Flachzuschnitt einer Schutzhülle 1 einer achten Variante der Schutzhülle 1 nach der sechsten Ausführungsform. Diese Schutzhülle 1 der achten Variante unterscheidet sich von der Schutzhülle 1 der sechsten Ausführungsform und der Schutzhülle 1 der ersten bis siebten Varianten dahingehend, dass nur noch eine durchgängig ausgebildete (nicht-lineare) Perforationslinie 29 vorgesehen ist. Die Perforationslinie 29 ist in etwa pilzförmig, in etwa nach der Art eines stilisierten Champignons, ausgebildet. Sie erstreckt sich mit einem ersten Ende ausgehend von der der Klappfalzlinie 4 gegenüberliegenden kurzen Seitenkante der Deckplatte 3 nahezu parallel zu den langen Seitenkanten der Deckplatte 3. Die Perforationslinie 29 erstreckt sich mit einem zweiten Ende ausgehend von der der Klappfalzlinie 4 gegenüberliegenden kurzen Seitenkante der Deckplatte 3 nahezu parallel zu den langen Seitenkanten der Deckplatte 3 und nahezu parallel zum ersten Ende. Erstes und zweites Ende der Perforationslinie 29 sind um die Breite der Lasche 6 voneinander beabstandet. Zwischen erstem und zweiten Ende der Perforationslinie 29 erstreckt sich eine pilzkopfartig geformte Linie. Die Perforationslinie 29 ist achsensymmetrisch (zu einer die kurzen Seitenkanten der Deckplatte 3 halbierenden Linie) auf der Deckplatte 3 angeordnet.

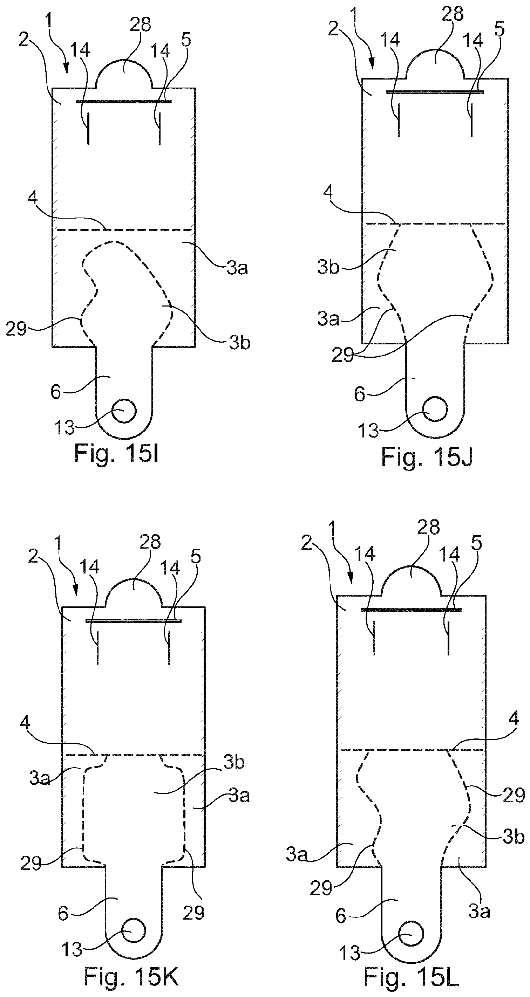

Fig. 15I ist eine Draufsicht auf einen Flachzuschnitt einer Schutzhülle 1 einer neunten Variante der Schutzhülle 1 nach der sechsten Ausführungsform. Auch bei der Schutzhülle 1 der neunten Variante ist wie bei der Schutzhülle 1 der achten Variante nur eine (durchgängig verlaufende und nicht-lineare) Perforationslinie 29 vorgesehen. Hier ist die Perforationslinie 1 nicht achsensymmetrisch ausgebildet. Die Perforationslinie 29 verläuft mit mehreren Abkrümmungen und Kurven. Beiden Enden der Perforationslinie 29 münden in die / enden an der Klappfalzlinie 4 gegenüberliegenden kurze Seitenkante der Deckplatte 3 und sind an dieser Stelle um die Breite der Lasche 6 voneinander beabstandet.

Fig. 15J ist eine Draufsicht auf einen Flachzuschnitt einer Schutzhülle 1 einer zehnten Variante der Schutzhülle 1 nach der sechsten Ausführungsform. Hier sind wieder, wie bei der Schutzhülle 1 der sechsten Ausführungsform und bei der Schutzhülle 1 der ersten bis siebten Variante zwei (nicht-lineare) Perforationslinien 29 vorgesehen. Diese erstrecken sich jeweils mit einem Abstand von den einander gegenüberliegenden Enden der Klappfalzlinie 4 ausgehend von der Klappfalzlinie 4 zu der der Klappfalzlinie 4 gegenüberliegenden kurzen Seitenkante der Deckplatte 3. Die beiden Perforationslinien 29 bilden zusammen in etwa eine bauchige Vasenform ab. Die Perforationslinien 29 sind achsensymmetrisch (in Bezug auf eine die kurzen Seitenkanten der Deckplatte 3 halbierende Line) zueinander ausgebildet. An der Stelle, an der die beiden Perforationslinien 29 in die der Klappfalzlinie 4 gegenüberliegende kurze Seitenkante der Deckplatte 3 münden, sind sie um die Breite der Lasche 6 voneinander beabstandet.

Fig. 15K ist eine Draufsicht auf einen Flachzuschnitt einer Schutzhülle 1 einer elften Variante der Schutzhülle 1 nach der sechsten Ausführungsform. Dabei erstrecken sich die (hauptsächlich linear, teilweise nicht-linear ausgebildeten) Perforationslinien 29 der Schutzhülle 1 der elften Variante ähnlich zu den Perforationslinien der Schutzhülle 1 der zehnten Variante. Allerdings bilden die beiden Perforationslinien 29 hier keine bauchige Vasenform aus, sondern verlaufen großteils / hauptsächlich parallel zu bzw. entlang der langen Seitenkanten der Deckplatte 3. Dabei sind die Perforationslinien 29 jeweils von den langen Seitenkanten der Deckplatte 3 beabstandet. Die Perforationslinien 29 sind achsensymmetrisch (in Bezug auf eine die kurzen Seitenkanten der Deckplatte 3 halbierende Line) zueinander ausgebildet. An der Stelle, an der die beiden Perforationslinien 29 in die der Klappfalzlinie 4 gegenüberliegende kurze Seitenkante der Deckplatte 3 münden, sind sie um die Breite der Lasche 6 voneinander beabstandet.

Fig. 15L ist eine Draufsicht auf einen Flachzuschnitt einer Schutzhülle 1 einer zwölften Variante der Schutzhülle 1 nach der sechsten Ausführungsform. Hier sind wieder zwei (nicht-lineare) Perforationslinien 29 vorgesehen. Die beiden Perforationslinien 29 verlaufen geschwungen bzw. in Wellenform. Die beiden Perforationslinien 29 verlaufen mit zueinander unterschiedlich geschwungenen Linien und sind nicht symmetrisch zueinander ausgebildet. An der Stelle, an der die beiden Perforationslinien 29 in die der Klappfalzlinie 4 gegenüberliegende kurze Seitenkante der Deckplatte 3 münden, sind sie um die Breite der Lasche 6 voneinander beabstandet.

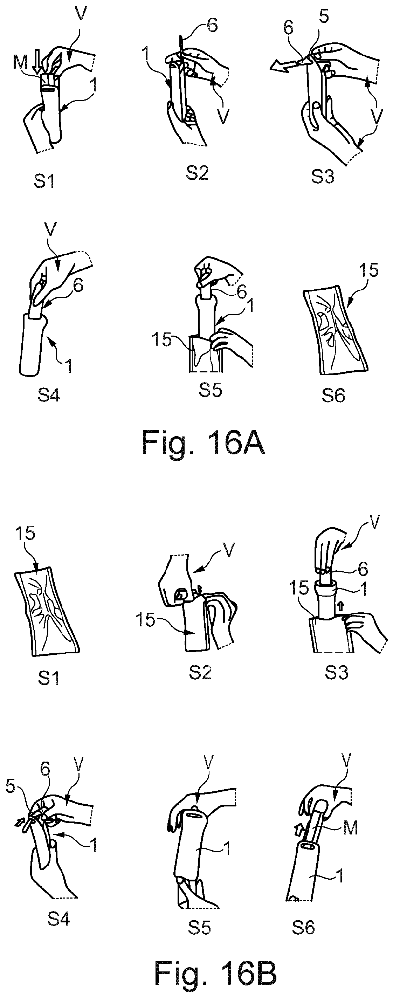

Fig. 16A zeigt schematisch die Prozessschritte S1 bis S7 zum Einführen eines Medizinprodukts M in eine offenbarungsgemäße Verpackung mit taschenförmiger Schutzhülle 1 und Sterilverpackung 15. Im Schritt S1 wird zunächst das Medizinprodukt M in die bereits zur Taschenform gefaltete Schutzhülle 1 eingeführt. Im Schritt S2 wird die Lasche 6 der Schutzhülle 1, in der das Medizinprodukt M angeordnet ist, vom Verwender V gegriffen. Im Schritt S3 wird die Lasche 6 an Basisplatte 2 oder Deckplatte 3 in die entsprechende Aufnahme 5 an der anderen aus Basisplatte 2 oder Deckplatte 3 eingeführt. In Schritt S4 wird die Lasche 6 vollständig in gerade gezogen und der Verwender V greift die Schutzhülle 1 an der Lasche 6. In Schritt S5 wird die Schutzhülle 1 in die Sterilverpackung 15 eingeführt. Dabei wird die Schutzhülle 1 an der Lasche 6 gehalten und geführt. Die Schutzhülle 1 wird so in der Sterilverpackung 15 angeordnet, dass sie vollständig von der Sterilverpackung 15 aufgenommen ist. Im Schritt S6 wird die Sterilverpackung 15 mit der darin angeordneten Schutzhülle 1, in der wiederum das Medizinprodukt M angeordnet ist, vakuumiert. Nach Schritt S6 ist das Verpackungssystem für den sterilen Transport bereit.

Fig. 16B zeigt die Prozessschritte S1 bis S6 zum Auspacken des Medizinprodukts M aus der offenbarungsgemäßen Verpackung. In Schritt S1 ist nochmals die vakuumierte Sterilverpackung 15 dargestellt, in welcher die Schutzhülle 1 angeordnet ist, in der wiederum das Medizinprodukt M angeordnet ist (wie in S6 der Fig. 15A). In Schritt S2 wird die Sterilverpackung 15, insbesondere an einer perforierten Stelle, manuell geöffnet bzw. aufgerissen. Im Schritt S3 wird die Schutzhülle 1 aus der Sterilverpackung 15 gezogen. Dabei wird die Schutzhülle 1 an der Lasche 6 gegriffen. Im Schritt S4 wird die Lasche 6 aus der Aufnahme 5 gezogen. Im Schritt S5 greift der Verwender V in die Schutzhülle 1, die hier noch in Taschenform gefaltet ist, um das in der Schutzhülle 1 angeordnete Medizinprodukt M zu greifen. Im Schritt S6 zieht der Verwender V das Medizinprodukt M aus der Schutzhülle 1.

Wenn in dieser Anmeldung beschrieben ist, dass sich die Perforationslinien 29 an die bzw. von der Klappfalzlinie 4 erstrecken, schließt dies den Fall ein, dass die Perforationslinien 29 in der Nähe der Klappfalzlinie 4 enden, d.h. bis zu 1cm, bevorzugt bis zu 0,5cm, von der Klappfalzlinie 4 entfernt enden bzw. beginnen. Dies gilt insbesondere für die Ausführungsformen der Fig. 12 bis 14, 15A bis 15F und 15J bis 15L.

Die Aspekte der verschiedenen Ausführungsformen können miteinander kombiniert werden, solange dies technisch möglich und sinnvoll ist.

### Bezugszeichenliste

- 1: Schutzhülle
- 2: Basisplatte
- 3: Deckplatte
- 3a: Verbindeabschnitt der Deckplatte
- 3b: Aufreißabschnitt der Deckplatte
- 4: Klappfalzlinie (zwischen Basis- und Deckplatte)
- 5: Aufnahme
- 6: Lasche
- 7: erste Seitenplatte
- 8: zweite Seitenplatte
- 9: Schwenkfalzlinie (zwischen Basisplatte und erster Seitenplatte 7)
- 10: Schwenkfalzlinie (zwischen Basisplatte und zweiter Seitenplatte 8)
- 11: Einrastschlitz in erster Seitenplatte 7
- 12: Einrastschlitz in zweiter Seitenplatte 8
- 13: Aussparung
- 14: Schlitze in Basisplatte
- 15: Sterilverpackung bzw. Umverpackung
- 16: (schlitzartige) Aufnahme in erster Seitenplatte 7
- 17: (schlitzartige) Aufnahme in zweiter Seitenplatte 8
- 18: Schließlasche
- 19: Widerhaken
- 20: Einstecklasche
- 21: Laschenaufnahme
- 22, 23: erste Zusatzlaschen
- 24 bis 27: zweite Zusatzlaschen
- 28: Vorsprung
- 29: Perforationslinie
- 29.1 - 29.3: erster bis dritter Perforationslinienabschnitt
- M: Medizinprodukt
- P: Packmittel
- V: Verwender

## Patentansprüche

1. Verpackung für ein medizinisches Produkts (M) mit
einer eine Sterilbarriere ausbildenden Umverpackung (15), die einen versiegelten oder versiegelbaren Aufnahmeraum für das medizinische Produkt (M) ausbildet, und
einer Produktauflagerung und/oder Halterung zur kontaktfreien Lagerung des Produkts innerhalb der Umverpackung, wobei
die Produktauflagerung und/oder Halterung eine, insbesondere taschenförmige, Schutzhülle (1) zur Aufnahme des medizinischen Produkts (M) und zur Beabstandung der Umverpackung vom Produkt ist, die aus einem einzigen, flexiblen Flachzuschnitt durch Falten entlang zumindest einer vordefinierten Klappfalzlinie (4) hergestellt ist, **dadurch gekennzeichnet, dass**
der Flachzuschnitt eine als Auflage für das medizinische Produkt (M) dienende Basisplatte (2), die eine Basis-Eingriffseinrichtung (5/6) hat, und eine Klapp- oder Deckplatte (3) aufweist, die mittels der Klappfalzlinie (4) mit der Basisplatte (2) gekoppelt ist und in einer in Richtung zur Basisplatte (2) umgeklappten Position mit der Basis-Eingriffseinrichtung (5/6) zum Halten der umgeklappten Position in Wirkeingriff bringbar ist, sodass die in einem Geschlossenzustand aufeinanderliegenden Stirnseiten von Basisplatte (2) und Klapp- oder Deckplatte (3), die der Klappfalzlinie (4) jeweils gegenüberliegen, verschlossen sind, und
die Schutzhülle (2) in einem durch Falten erzeugten Geschlossenzustand nach allen Seiten hin zur Umverpackung (15) hin verschlossen ist.

2. Verpackung nach Anspruch 1, **dadurch gekennzeichnet, dass** der Flachzuschnitt weiterhin eine erste Seitenplatte (7) aufweist, die über eine senkrecht zur Klappfalzlinie (4) ausgerichtete Schwenkfalzlinie (9) mit der Basisplatte (2) gekoppelt ist und eine Seiten-Eingriffseinrichtung zum Halten der ersten Seitenplatte (7) in einer zur Basisplatte (2) hin geschwenkten Position hat.

3. Verpackung nach Anspruch 2, **dadurch gekennzeichnet, dass** der Flachzuschnitt weiterhin eine zweite Seitenplatte (8) aufweist, die über eine senkrecht zur Klappfalzlinie (4) ausgerichtete Schwenkfalzlinie (10) mit der Basisplatte (2) gegenüberliegend zur ersten Seitenplatte (7) gekoppelt ist und eine Seiten-Eingriffseinrichtung zum Halten der zweiten Seitenplatte (8) in einer zur Basisplatte (2) hin geschwenkten Position hat.

4. Verpackung nach Anspruch 3, **dadurch gekennzeichnet, dass** die jeweilige Seiten-Eingriffsvorrichtung der ersten Seitenplatte (7) und der zweiten Seitenplatte (8) als Einrastschlitz (11, 12) ausgebildet ist, über den die erste Seitenplatte (7) und die zweite Seitenplatte (8) miteinander verhakbar sind, oder die jeweilige Seiten-Eingriffsvorrichtung der ersten Seitenplatte (7) und der zweiten Seitenplatte (8) als, insbesondere schlitzförmige, Aufnahme (16, 17) ausgebildet ist, in welche ein laschenartiger Abschnitt (6) der Klapp- oder Deckplatte (3) einführbar ist.

5. Verpackung nach Anspruch 1, **dadurch gekennzeichnet, dass** der Flachzuschnitt weiterhin eine Schließlasche (18), die über eine parallel zur Klappfalzlinie (4) ausgerichtete Schwenkfalzlinie mit der Basisplatte (2) oder der Klapp-oder Deckplatte (3) gekoppelt ist, und eine Einstecklasche (20) aufweist, die über eine parallel zur Klappfalzlinie (4) ausgerichtete Schwenkfalzlinie mit derjenigen aus Basisplatte (2) und Klapp- oder Deckplatte (3) gekoppelt ist, die nicht mit der Schließlasche (18) gekoppelt ist, und zwischen sich und der mit ihr gekoppelten aus Basisplatte (2) oder Klapp- oder Deckplatte (3) eine, insbesondere schlitzförmige, Laschenaufnahme (21) zum Aufnehmen der Schließlasche (18) ausbildet.

6. Verpackung nach Anspruch 1, **dadurch gekennzeichnet, dass** der Flachzuschnitt jeweils eine erste Zusatzlasche (22, 23) an der Basisplatte (2) und an der Klapp-oder Deckplatte (3) aufweist, die an die zueinander benachbarten und senkrecht zur Klappfalzlinie (4) verlaufenden Seitenkanten der Basisplatte (2) bzw. der Klapp- oder Deckplatte (3) angrenzen und im Geschlossenzustand zusammen ein Zusatzfach zur Aufnahme eines weiteren Produkts (M, P) ausbilden.

7. Verpackung nach Anspruch 6, **dadurch gekennzeichnet, dass** der Flachzuschnitt weiterhin weitere zweite, insbesondere jeweils zwei, Zusatzlaschen (24 bis 27) an der Basisplatte (2) und an der Klapp- oder Deckplatte (3) aufweist, die jeweils an eine von der senkrecht zur Klappfalzlinie (4) verlaufenden Seitenkante der Basisplatte (2) bzw. der Klapp- oder Deckplatte (3) parallel beabstandeten Seitenkante der ersten Zusatzlasche (22, 23) angrenzen und die zweiten Zusatzlaschen (24 bis 27) an der Basisplatte (2) bzw. der Klapp- oder Deckplatte (3) voneinander beabstandet sind und im Geschlossenzustand zusammen mit der über- bzw. unter ihr liegenden zweiten Zusatzlasche (24 bis 27) an der anderen aus Basisplatte (2) und Klapp- oder Deckplatte (3) weitere Zusatzfächer zur Aufnahme weiterer Produkte (M, P) ausbilden.

8. Verpackung nach Anspruch 1, **dadurch gekennzeichnet, dass** die Klapp- oder Deckplatte (3) in einen Verbindeabschnitt (3a) und in einen Aufreißabschnitt (3b) unterteilt ist, der über zumindest eine Perforationslinie (29) von dem Verbindeabschnitt (3a) getrennt ist, die eine Sollbruchstelle zwischen Verbindeabschnitt (3a) und Aufreißabschnitt (3b) ausbildet.

9. Verpackung nach Anspruch 8, **dadurch gekennzeichnet, dass** ein erstes Ende der zumindest einen Perforationslinie (29) und ein zweites Ende derselben Perforationslinie (29) oder, im Falle zweier Perforationslinien (29) an derselben Klapp-oder Deckplatte (3), eine erste Perforationslinie (29) und eine zweite Perforationslinie (29) in eine der Klappfalzlinie (4) gegenüberliegende Seitenkante der Klapp- oder Deckplatte (3) münden.

10. Verpackung nach Anspruch 8 oder 9, **dadurch gekennzeichnet, dass** sich ausgehend von der der Klappfalzlinie (4) gegenüberliegenden Seitenkante der Klapp-oder Deckplatte (3) ein laschenartiger Abschnitt (6) der Klapp- oder Deckplatte (3) von der Klapp- oder Deckplatte (3) weg erstreckt, der mit der Basis-Eingriffseinrichtung an der Basisplatte (2) in Wirkeingriff bringbar ist und der stoffeinstückig mit dem Aufreißabschnitt (3b) ausgebildet ist.

11. Verpackung nach Anspruch 9 und 10, **dadurch gekennzeichnet, dass** das erste Ende der zumindest einen Perforationslinie (29) und das zweite Ende derselben Perforationslinie (29) oder, im Falle zweier Perforationslinien (29) an derselben Klapp-oder Deckplatte (3), die erste Perforationslinie (29) und die zweite Perforationslinie (29) an der Stelle, an der sie jeweils in die der Klappfalzlinie (4) gegenüberliegende Seitenkante der Klapp- oder Deckplatte (3) münden, um die Breite des laschenartigen Abschnittes (6) voneinander beabstandet sind.

12. Verpackung nach einem der Ansprüche 8 bis 11, **dadurch gekennzeichnet, dass** zwei Perforationslinien (29) vorgesehen sind, die nicht-linear ausgebildet sind, in Bezug auf eine die Klappfalzlinie (4) halbierende Linie achsensymmetrisch zueinander angeordnet sind und sich jeweils ausgehend von der Klappfalzlinie (4) zu der der Klappfalzlinie (4) gegenüberliegenden Seitenkante der Klapp- oder Deckplatte (3) durchgängig erstrecken.

13. Verpackung nach einem der Ansprüche 1 bis 12, **dadurch gekennzeichnet, dass** die Basis-Eingriffseinrichtung der Basisplatte (2) als, insbesondere schlitzförmige, Aufnahme (5) ausgebildet ist, die in unmittelbarer Nähe zu einer der Seitenkanten der Basisplatte (2) angeordnet ist, und die Klapp- oder Deckplatte (3) einen laschenförmigen Abschnitt (6) aufweist, der sich von der Seitenkante der Klapp- oder Deckplatte (3) nach außen erstreckt, die im Geschlossenzustand der Seitenkante mit Aufnahme (5) der Basisplatte (2) gegenüberliegt, und der in die Aufnahme (5) der Basisplatte (2) einführbar ist, oder
dass die Basis-Eingriffseinrichtung der Basisplatte (2) als laschenförmiger Abschnitt (6) der Basisplatte (2) ausgebildet ist und die Klapp- oder Deckplatte (3) eine im Geschlossenzustand diesen laschenförmigen Abschnitt (6) der Basisplatte (2) aufnehmende Aufnahme (5) aufweist.

14. Verpackung nach einem der Ansprüche 1 bis 13, **dadurch gekennzeichnet, dass** die Schutzhülle (1) beziehungsweise der sie ausbildende Flachzuschnitt aus einem flexiblen, abriebfesten und sterilisierbaren Material, vorzugsweise aus TPU, Tyvek, Silikon oder Kunststoffen mit vergleichbaren Eigenschaften, hergestellt ist.

## Claims

1. A packaging for medical product (M) with
a secondary packaging (15) forming a sterile barrier, which forms a sealed or sealable receiving space for the medical product (M), and
a product storage space and/or holder for contact-free storage of the product within the secondary packaging, wherein
the product storage space and/or holder is an, in particular pocket-shaped, protective cover (1) for receiving the medical product (M) and for interspacing the secondary packaging of the product, which is made of a single, flexible flat cut by folding along at least one predefined hinged fold line (4),
**characterized in that**
the flat cut comprises a base plate (2) serving as a storage space for the medical product (M) and having a base latching device (5/6), and a folding or cover plate (3), which is coupled to the base plate (2) via the hinged fold line (4) and can be brought into operative engagement with the base latching device (5/6) in a position where the folding or cover plate (3) is folded toward/ onto the base plate (2) to hold the folded position, so that the front sides of the base plate (2) and the folding or cover plate (3), which lie opposite each other in a closed state and which are respectively opposite the hinged fold line (4), are closed, and
the protective cover (2) is closed on all sides toward the secondary packaging (15) in a closed state produced by folding.

2. The packaging according to claim 1, **characterized in that** the flat cut comprises furthermore:
- a first side plate (7), which is coupled to the base plate (2) via a swivel fold line (9) aligned perpendicular to the hinged fold line (4) and has a side latching device for holding the first side plate (7) in a position pivoted toward the base plate (2).

3. The packaging according to claim 2, **characterized in that**
the flat cut further comprises a second side plate (8) which is coupled to the base plate (2) opposite the first side plate (7) via a swivel fold line (10) aligned perpendicularly to the hinged fold line (4) and which has a side latching device for holding the second side plate (8) in a position pivoted toward the base plate (2).

4. The packaging according to claim 3, **characterized in that**
the respective side engagement device of each of the first side plate (7) and the second side plate (8) is configured as a latching slit (11, 12), via which the first side plate (7) and the second side plate (8) can be hooked together, or the respective side engagement device of the first side plate (7) and of the second side plate (8) is configured as a receptacle (16, 17), in particular a slit-like receptacle, into which a tab-like portion (6) of the folding or cover plate (3) can be inserted.

5. The packaging according to claim 1, **characterized in that** the flat cut furthermore comprises:
- a closing tab (18), which is coupled to the base plate (2) or the folding or cover plate (3) via a swivel fold line aligned parallel to the hinged fold line (4), and
- an insert tab (20) which is coupled via a swivel fold line aligned parallel to the hinged fold line (4) to that of the base plate (2) and folding or cover plate (3) which is not coupled to the closing tab (18), and forms between itself and the base plate (2) or folding or cover plate (3) coupled thereto a, in particular slit-like, tab receptacle (21) for receiving the closing tab (18).

6. The packaging according to claim 1, **characterized in that** the flat cut comprises:
- in each case a first additional tab (22, 23) on the base plate (2) and on the folding or cover plate (3), which adjoin the side edges of the base plate (2) and of the folding or cover plate (3) which are adjacent to one another and extend perpendicularly to the hinged fold line (4) and, in the closed state, together form an additional compartment for receiving a further product (M, P).

7. The packaging according to claim 6, **characterized in that** the flat cut furthermore has further second, in particular in each case two, additional tabs (24 to 27) on the base plate (2) and on the folding or cover plate (3), which in each case adjoin a side edge of the first additional tab (22, 23), and the second additional tabs (24 to 27) on the base plate (2) or the folding or cover plate (3) are spaced apart from one another and, in the closed state, together with the second additional tab (24 to 27) lying above or below it, form further additional compartments for receiving further products (M, P) on the other of the base plate (2) and the folding or cover plate (3).

8. The packaging according to claim 1, **characterized in that**
the folding or cover plate (3) is divided into a connecting portion (3a) and a tear-open portion (3b), which is separated from the connecting portion (3a) by at least one perforation line (29), which forms a predetermined breaking point between the connecting portion (3a) and the tear-open portion (3b).

9. The packaging according to claim 8, **characterized in that** a first end of the at least one perforation line (29) and a second end of the same perforation line (29) or, in the case of two perforation lines (29) on the same folding or cover plate (3), a first perforation line (29) and a second perforation line (29) open into a side edge of the folding or cover plate (3) opposite the hinged fold line (4).

10. The packaging according to claim 8 or 9, **characterized in that**, starting from the side edge of the folding or cover plate (3) opposite the hinged fold line (4), a tab-like portion (6) of the folding or cover plate (3) extends away from the folding or cover plate (3), which can be brought into operative engagement with the base latching device (5/6) on the base plate (2) and which is integrally configured with the tear-open portion (3b).

11. The packaging according to claims 9 and 10, **characterized in that** the first end of the at least one perforation line (29) and the second end of the same perforation line (29) or, in the case of two perforation lines (29) on the same folding or cover plate (3), the first perforation line (29) and the second perforation line (29) are spaced apart by the width of the tab-like portion (6) at the point at which they each open into the side edge of the folding or cover plate (3) opposite the hinged fold line (4).

12. The packaging according to one of claims 8 to 11, **characterized in that** two perforation lines (29) are provided which are configured non-linearly and are arranged axially symmetrically to one another with respect to a line bisecting the hinged fold line (4) and extend continuously in each case starting from the hinged fold line (4) to the side edge of the folding or cover plate (3) opposite the hinged fold line (4).

13. The packaging according to one of claims 1 to 12, **characterized in that** the base latching device (5/6) of the base plate (2) is configured as a, in particular slit-like, receptacle (5), which is arranged in the immediate vicinity of one of the side edges of the base plate (2), and the folding or cover plate (3) has a tab-shaped portion (6) which extends outward from the side edge of the folding or cover plate (3), which in the closed state is opposite the side edge with receptacle (5) of the base plate (2), and which is insertable into the receptacle (5) of the base plate (2), or
**in that** the base latching device (5/6) of the base plate (2) is configured as a tab-shaped portion (6) of the base plate (2) and the folding or cover plate (3) has a receptacle (5) which receives this tab-shaped portion (6) of the base plate (2) in the closed state.

14. The packaging according to one of claims 1 to 13, **characterized in that** the protective cover (1) or the flat cut forming it is made of a flexible, abrasion-resistant and sterilizable material, preferably TPU, Tyvek, silicone or plastics with comparable properties.

## Revendications

1. Emballage pour un produit médical (M) avec un suremballage (15) formant une barrière stérile qui forme un espace de réception scellé ou scellable pour le produit médical (M), et
un espace de stockage de produit et/ou support pour le logement sans contact du produit dans le suremballage, dans lequel
l'espace de stockage de produit et/ou le support est une enveloppe de protection (1) en particulier en forme de poche pour la réception du produit médical (M) et pour l'espacement du suremballage du produit qui est fabriqué à partir d'une seule découpe plate flexible par pliage le long d'au moins une ligne de pliage articulée (4) prédéfinie, **caractérisé en ce que**
la découpe plate présente une plaque de base (2) servant d'appui pour le produit médical (M), plaque qui a un dispositif de prise de base (5/6), et une plaque de rabattement ou de recouvrement (3) qui est couplée au moyen d'une ligne de pliage articulée (4) à la plaque de base (2) et peut être amenée en prise active dans une position rabattue en direction de la plaque de base (2) avec le dispositif de prise de base (5/6) pour le maintien de la position rabattue de sorte que les côtés avant reposant l'un sur l'autre dans un état fermé de la plaque de base (2) et de la plaque de rabattement ou de recouvrement (3) qui font face respectivement à la ligne de pliage articulée (4), soient fermés et
l'enveloppe de protection (2) est fermée dans un état fermé généré par pliage de tous les côtés vers le suremballage (15).

2. Emballage selon la revendication 1, **caractérisé en ce que** la découpe plate présente en outre une première plaque latérale (7) qui est couplée par le biais d'une ligne de pliage pivotante (9) orientée perpendiculairement à la ligne de pliage articulée (4) à la plaque de base (2) et a un dispositif de prise latérale pour le maintien de la première plaque latérale (7) dans une position pivotée vers la plaque de base (2).

3. Emballage selon la revendication 2, **caractérisé en ce que** la découpe plate présente en outre une seconde plaque latérale (8) qui est couplée par le biais d'une ligne de pliage pivotante (10) orientée perpendiculairement à la ligne de pliage articulée (4) à la plaque de base (2) en face de la première plaque latérale (7) et a un dispositif de prise latérale pour le maintien de la seconde plaque latérale (8) dans une position pivotée vers la plaque de base (2).

4. Emballage selon la revendication 3, **caractérisé en ce que** le dispositif de prise latérale respective de la première plaque latérale (7) et de la seconde plaque latérale (8) est formé comme fente d'encliquetage (11, 12), par le biais de laquelle la première plaque latérale (7) et la seconde plaque latérale (8) sont accrochables entre elles, ou le dispositif de prise latérale respectif de la première plaque latérale (7) et de la seconde plaque latérale (8) est formé comme logement (16, 17) en particulier en fente, dans lequel une section (6) de type languette de la plaque de rabattement ou de recouvrement (3) peut être introduite.

5. Emballage selon la revendication 1, **caractérisé en ce que** la découpe plate présente en outre une languette de fermeture (18) qui est couplée par le biais d'une ligne de pliage pivotante orientée parallèlement à la ligne de pliage articulée (4) à la plaque de base (2) ou la plaque de rabattement ou de recouvrement (3), et une languette d'enfichage (20) qui est couplée par le biais d'une ligne de pliage pivotante orientée parallèlement à la ligne de pliage articulée (4) à celle composée de la plaque de base (2) et de la plaque de rabattement ou de recouvrement (3) qui n'est pas couplée à la languette de fermeture (18), et entre elles et celle couplée avec elle composée de la plaque de base (2) ou de la plaque de rabattement ou de recouvrement (3), forme un logement de languette (21) en particulier en fente pour la réception de la languette de fermeture (18).

6. Emballage selon la revendication 1, **caractérisé en ce que** la découpe plate présente respectivement une première languette supplémentaire (22, 23) au niveau de la plaque de base (2) et au niveau de la plaque de rabattement ou de recouvrement (3) qui sont contiguës aux arêtes latérales contiguës les unes aux autres et s'étendant perpendiculairement à la ligne de pliage articulée (4) de la plaque de base (2) ou de la plaque de rabattement ou de recouvrement (3) et forment dans l'état fermé ensemble un compartiment supplémentaire pour le logement d'un autre produit (M, P).

7. Emballage selon la revendication 6, **caractérisé en ce que** la découpe plate présente en outre d'autres secondes, en particulier respectivement deux, languettes supplémentaires (24 à 27) au niveau de la plaque de base (2) et au niveau de la plaque de rabattement ou de recouvrement (3) qui sont contiguës respectivement à une arête latérale espacée parallèlement de l'arête latérale s'étendant perpendiculairement à la ligne de pliage articulée (4) de la plaque de base (2) ou de la plaque de rabattement ou de recouvrement (3) de la première languette supplémentaire (22, 23) et les secondes languettes supplémentaires (24 à 27) sont espacées les unes des autres au niveau de la plaque de base (2) ou de la plaque de rabattement ou de recouvrement (3) et forment dans l'état fermé conjointement avec la seconde languette supplémentaire (24 à 27) se trouvant sur ou sous elle au niveau de l'autre composée de la plaque de base (2) et de la plaque de rabattement ou de recouvrement (3) d'autres compartiments supplémentaires pour le logement d'autres produits (M, P).

8. Emballage selon la revendication 1, **caractérisé en ce que** la plaque de rabattement ou de recouvrement (3) est divisée en une section de liaison (3a) et en une section de rupture (3b) qui est séparée par le biais d'au moins une ligne de perforation (29) de la section de liaison (3a) qui forme un point destiné à la rupture entre la section de liaison (3a) et la section de rupture (3b).

9. Emballage selon la revendication 8, **caractérisé en ce qu'**une première extrémité de l'au moins une ligne de perforation (29) et une seconde extrémité de la même ligne de perforation (29) ou dans le cas de deux lignes de perforation (29) au niveau de la même plaque de rabattement ou de recouvrement (3), une première ligne de perforation (29) et une seconde ligne de perforation (29) débouchent dans une arête latérale opposée à la ligne de pliage articulée (4) de la plaque de rabattement ou de recouvrement (3).

10. Emballage selon la revendication 8 ou 9, **caractérisé en ce qu'**à partir de l'arête latérale opposée à la ligne de pliage articulée (4) de la plaque de rabattement ou de recouvrement (3), une section (6) de type languette de la plaque de rabattement ou de recouvrement (3) s'étend depuis la plaque de rabattement ou de recouvrement (3) qui peut être amenée en prise active avec le dispositif de prise de base au niveau de la plaque de base (2) et qui est formée d'un seul tenant en matière avec la section de rupture (3b).

11. Emballage selon les revendications 9 et 10, **caractérisé en ce que** la première extrémité de l'au moins une ligne de perforation (29) et la seconde extrémité de la même ligne de perforation (29) ou dans le cas de deux lignes de perforation (29) au niveau de la même plaque de rabattement ou de recouvrement (3), la première ligne de perforation (29) et la seconde ligne de perforation (29) sont espacées à l'endroit auquel elles débouchent respectivement dans l'arête latérale opposée à la ligne de pliage articulée (4) de la plaque de rabattement ou de recouvrement (3) de la largeur de la section (6) de type languette.

12. Emballage selon l'une quelconque des revendications 8 à 11, **caractérisé en ce que** deux lignes de perforation (29) sont prévues, lesquelles sont formées de manière non linéaire, sont agencées de manière symétrique à l'axe l'une par rapport à l'autre par rapport à une ligne divisant en deux la ligne de pliage articulée (4) et s'étendent respectivement en continu à partir de la ligne de pliage articulée (4) vers l'arête latérale opposée à la ligne de pliage articulée (4) de la plaque de rabattement ou de recouvrement (3).

13. Emballage selon l'une quelconque des revendications 1 à 12, **caractérisé en ce que** le dispositif de prise de base de la plaque de base (2) est formé comme logement (5) en particulier en fente qui est agencé à proximité immédiate d'une des arêtes latérales de la plaque de base (2), et la plaque de rabattement ou de recouvrement (3) présente une section (6) en forme de languette qui s'étend vers l'extérieur depuis l'arête latérale de la plaque de rabattement ou de recouvrement (3) qui fait face dans l'état fermé de l'arête latérale avec le logement (5) à la plaque de base (2), et qui peut être introduite dans le logement (5) de la plaque de base (2) ou
le dispositif de prise de base de la plaque de base (2) est formé comme section (6) en forme de languette de la plaque de base (2) et la plaque de rabattement ou de recouvrement (3) présente un logement (5) recevant dans l'état fermé cette section (6) en forme de languette de la plaque de base (2).

14. Emballage selon l'une quelconque des revendications 1 à 13, **caractérisé en ce que** l'enveloppe de protection (1) ou la découpe plate la formant est fabriquée en un matériau flexible, résistant à l'usure et stérilisable, de préférence en TPU, Tyvek, silicone ou matières plastiques aux propriétés comparables.
